(19) Europäisches Patentamt
European Patent Office
Office européen des brevets

(11) Publication number: **0 327 500 A2**

# (12) EUROPEAN PATENT APPLICATION

(21) Application number: 89810063.1

(22) Date of filing: 25.01.89

(51) Int. Cl.[4]: C 07 D 471/04
C 07 F 7/18, C 07 F 9/65,
A 61 K 31/435
//(C07D471/04,231:00,221:00)

(30) Priority: 27.01.88 US 149232

(43) Date of publication of application:
09.08.89 Bulletin 89/32

(84) Designated Contracting States:
AT BE CH DE FR GB IT LI LU NL SE

(71) Applicant: SANDOZ AG
Lichtstrasse 35
CH-4002 Basel (CH)

(84) Designated Contracting States:
BE CH FR GB IT LI LU NL SE

(71) Applicant: SANDOZ-PATENT-GMBH
Humboldtstrasse 3
D-7850 Lörrach (DE)

(84) Designated Contracting States: DE

(71) Applicant: SANDOZ-ERFINDUNGEN
Verwaltungsgesellschaft m.b.H.
Brunner Strasse 59
A-1235 Wien (AT)

(84) Designated Contracting States: AT

(72) Inventor: Kathawala, Faizulla Gulamhusein
39 Woodland Avenue
Mountain Lakes, NJ 07946 (US)

(54) **5-Substituted 1H-pyrazolo[3,4-b]pyridine derivatives, their preparation and pharmaceutical compositions containing them.**

(57) Compounds of the formula

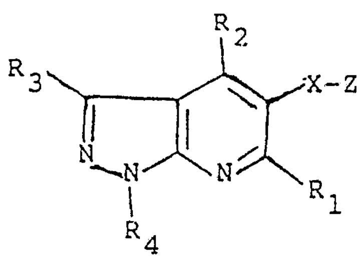

wherein the substituents have various significances are described, together with processes for their synthesis and intermediates, pharmaceutical compositions comprising such compounds and the use of such compounds for inhibiting cholesterol biosynthesis and lowering the blood cholesterol level and, therefore, their use in the treatment of hyperlipoproteinemia and atherosclerosis.

EP 0 327 500 A2

**Description**

## 5-SUBSTITUTED 1H-PYRAZOLO[3,4-b]PYRIDINE DERIVATIVES, THEIR PREPARATION AND PHARMACEUTICAL COMPOSITIONS CONTAINING THEM

This invention relates to 5-substituted 1H-pyrazolo[3,4-b]pyridine derivatives, in particular it concerns compounds of formula I

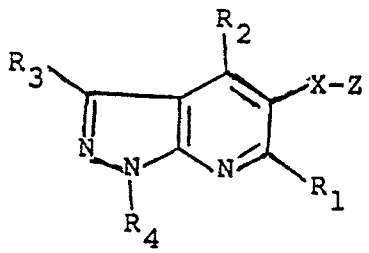

(I),

wherein $R_1$ is $C_{1-6}$alkyl not containing an asymmetric carbon atom, $C_{5-7}$cycloalkyl, ($C_{5-7}$cycloalkyl)methyl, phenyl-$(CH_2)_m$-, pyridyl-2, pyridyl-3, pyridyl-4, thienyl-2, thienyl-3 or Ring A

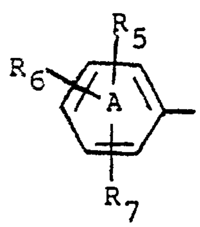

,

$R_2$ is $C_{1-6}$alkyl not containing an asymmetric carbon atom, $C_{5-7}$cycloalkyl, ($C_{5-7}$cycloalkyl)methyl, phenyl-$(CH_2)_m$-, pyridyl-2, pyridyl-3, pyridyl-4, thienyl-2, thienyl-3 or Ring B

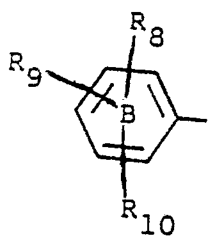

,

with the proviso that not more than one of $R_1$ and $R_2$ is a member of the group consisting of pyridyl-2, pyridyl-3, pyridyl-4, thienyl-2, thienyl-3, Ring A and Ring B,
$R_3$ is $C_{1-6}$alkyl not containing an asymmetric carbon atom, $C_{5-7}$cycloalkyl or Ring C

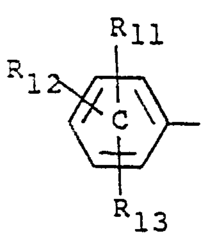

,

$R_4$ is $C_{1-6}$alkyl not containing an asymmetric carbon atom, $C_{5-7}$cycloalkyl or Ring D

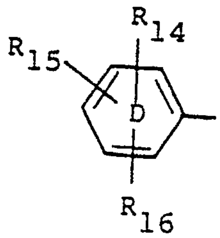 ,

X is $-(CH_2)_n-$, $-CH=CH-$, $-CH_2-CH=CH-$ or $-CH=CH-CH_2-$, wherein n is 1, 2 or 3, and

Z is 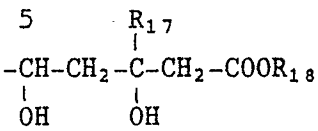 (a),

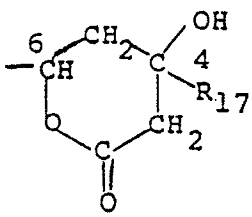 (b) or

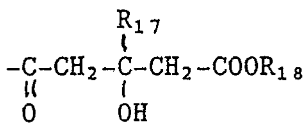 (c),

wherein $R_{17}$ is hydrogen or $C_{1-3}$alkyl, and $R_{18}$ is hydrogen, $R_{19}$ or M,
wherein $R_{19}$ is an ester group, and M is a cation,
with the proviso that Z may be a group of Formula c only when
(i) X is $-CH=CH-$ or $-CH_2-CH=CH-$ and/or
(ii) $R_{17}$ is $C_{1-3}$alkyl, wherein
each of $R_5$, $R_8$, $R_{11}$ and $R_{14}$ is independently hydrogen, $C_{1-3}$alkyl, n-butyl, i-butyl, t-butyl, $C_{1-3}$alkoxy, n-butoxy, i-butoxy, trifluoromethyl, fluoro, chloro, phenyl, phenoxy or benzyloxy,
each of $R_6$, $R_9$, $R_{12}$ and $R_{15}$ is independently hydrogen, $C_{1-3}$alkyl, $C_{1-3}$alkoxy, trifluoromethyl, fluoro, chloro, phenoxy or benzyloxy, and
each of $R_7$, $R_{10}$, $R_{13}$ and $R_{16}$ is independently hydrogen, $C_{1-2}$alkyl, $C_{1-2}$alkoxy, fluoro or chloro,
with the provisos that not more than one substituent on each of Rings A, B, C and D independently is trifluoromethyl, not more than one substituent on each of Rings A, B, C and D independently is phenoxy, and not more than one substituent on each of Rings A, B, C and D independently is benzyloxy, and
each m is independently 1, 2 or 3,
in free acid form, or in the form of an ester or δ-lactone thereof, or in salt form as appropriate, hereinafter referred to as "the compounds of the invention",
processes for the synthesis thereof, pharmaceutical compositions comprising them and their use for inhibiting cholesterol biosynthesis and lowering the blood cholesterol level and, therefore, in the treatment of hyperlipoproteinemia and atherosclerosis, and intermediates in the synthesis thereof.

Preferred esters are esters having a physiologically acceptable ester group. By the term "physiologically acceptable ester group" is meant a group which, together with the -COO- radical to which it is attached, forms an ester group which is physiologically acceptable. The preferred such groups are the physiologically acceptable and hydrolyzable ester groups. By the term "physiologically acceptable and hydrolyzable ester group" is meant a group which, together with the -COO-radical to which it is attached, forms an ester group which is physiologically acceptable and hydrolyzable under physiological conditions to yield a compound of Formula I wherein $R_{18}$ is hydrogen and an alcohol which itself is physiologically acceptable, i.e., non-toxic at

the desired dosage level, and which, preferably, is free of centers of asymmetry. Examples of such groups are $C_{1-3}$alkyl, n-butyl, i-butyl, t-butyl and benzyl, collectively referred to as $R'_{19}$.

When the compounds are in δ-lactone form Z is a group of formula b.

When the compounds are in salt form Z preferably is a group of Formula a or c and $R_{18}$ a group M, i.e. a cation. Preferred cations are pharmaceutically acceptable cations, especially sodium, potassium and ammonium.

For the avoidance of doubt, throughout this specification it is the right-hand side of the X radical that is attached to the Z group.

As is self-evident to those in the art, each compound of Formula I wherein Z is a group of Formula a or b has two centers of asymmetry (the two carbon atoms bearing the hydroxy groups in the group of Formula a and the carbon atom bearing the hydroxy group and the carbon atom having the free valence in the group of Formula b) and, therefore, there are four stereoisomeric forms (enantiomers) of each compound (two racemates or pairs of diastereoisomers), provided that $R_{18}$ does not contain any center of asymmetry. The four stereoisomers may be designated as the R,R, R,S, S,R and S,S enantiomers, all four stereoisomers being within the scope of this invention. When $R_{18}$ contains one or more centers of asymmetry, there are eight or more stereoisomers, all being within the scope of this invention. On the other hand, each compound of Formula I wherein Z is a group of Formula c has a single center of asymmetry (the carbon atom bearing the hydroxy group in the group of Formula c) and, therefore, there are two enantiomers of each compound, provided that $R_{18}$ does not contain any center of asymmetry. The two stereoisomers may be designated as the 3R and 3S enantiomers, both being within the scope of this invention. When $R_{18}$ contains one or more centers of asymmetry, there are four or more stereoisomers, all being within the scope of this invention. Since it is preferred that $R_{18}$ not contain a center of asymmetry and for reasons of simplicity, in both cases any additional stereoisomers resulting from the presence of one or more centers of asymmetry in $R_{18}$ will be ignored, it being assumed that $R_{18}$ is free of centers of asymmetry.

The compounds of Formula I may be divided into three groups, viz., those of Groups IA, IB and IC:

| | $R_1$ | $R_2$ |
|---|---|---|
| IA | Other than Ring A, pyridyl and thienyl | Ring B, pyridyl or thienyl |
| IB | Ring A, pyridyl or thienyl | Other than Ring B, pyridyl and thienyl |
| IC | Other than Ring A, pyridyl and thienyl | Other than Ring B, pyridyl and thienyl |

The compounds of each of Groups IA, IB and IC may be divided into three subgroups based upon the significance of Z, viz., Group IAa (the compounds of Group IA wherein Z is a group of Formula a), Group IAb (the compounds of Group IA wherein Z is a group of Formula b), Group IAc (the compounds of Group IA wherein Z is a group of Formula c), Group IBa (the compounds of Group IB wherein Z is a group of Formula a), Group IBb (the compounds of Group IB wherein Z is a group of Formula b), Group IBc (the compounds of Group IB wherein Z is a group of Formula c), Group ICa (the compounds of Group IC wherein Z is a group of Formula a), Group ICb (the compounds of Group IC wherein Z is a group of Formula b) and Group ICc (the compounds of Group IC wherein Z is a group of Formula c).

Preferably, one of $R_1$ and $R_2$ is $C_{1-6}$alkyl not containing an asymmetric carbon atom and the other is Ring A (if $R_1$) or Ring B (if $R_2$). Also preferably, at least one of $R_3$ and $R_4$ is $C_{1-6}$alkyl not containing an asymmetric carbon atom, more preferably $C_{1-3}$alkyl and most preferably $C_{1-2}$alkyl. More preferably, the preferences of both preceding sentences occur simultaneously. Thus, the preferred compounds of Formula I and the subscopes thereof are those having attaohed to the pyrazolopyridine ring i) Ring A or Ring B and (ii) three alkyl groups or Ring C or Ring D and two alkyl groups. The more preferred compounds of Formula I and the subscopes thereof are those having attached to the pyrazolopyridine ring Ring B and three alkyl groups or Rings B and C and two alkyl groups.

In Group IA:

$R_1$ is preferably $R_{1A}$, where $R_{1A}$ is $C_{1-6}$alkyl not containing an asymmetric carbon atom, more preferably $R'_{1A}$, where $R'_{1A}$ is $C_{1-4}$alkyl not containing an asymmetric carbon atom, even more preferably $R''_{1A}$, where $R''_{1A}$ is $C_{1-3}$alkyl, and most preferably i-propyl; and

$R_2$ is preferably $R_{2A}$, where $R_{2A}$ is Ring B, more preferably $R'_{2A}$, where $R_{2A}$ is Ring B wherein $R_8$ is $R'_8$, $R_9$ is $R'_9$, and $R_{10}$ is $R'_{10}$, even more preferably $R''_{2A}$, where $R''_{2A}$ is Ring B wherein $R_8$ is $R''_8$, $R_9$ is $R''_9$ and $R_{10}$ is hydrogen, and most preferably phenyl, 4-fluorophenyl or 3,5-dimethylphenyl, especially 4-fluorophenyl.

In Group IB:

$R_1$ is preferably $R'_{1B}$, where $R_{1B}$ is Ring A, more preferably $R'_{1B}$, where $R'_{1B}$ is Ring A wherein $R_5$ is $R'_5$, $R_6$ is $R'_6$, and $R_7$ is $R'_7$, even more preferably $R''_{1B}$, where $R''_{1B}$ is Ring A wherein $R_5$ is $R''_5$, $R_6$ is $R''_6$, and $R_7$ is

hydrogen, and most preferably phenyl, 4-fluorophenyl or 3,5-dimethylphenyl, especially 4-fluorophenyl; and $R_2$ is preferably $R_{2B}$, where $R_{2B}$ is $C_{1-6}$alkyl not containing an asymmetric carbon atom, more preferably $R'_{2B}$, where $R'_{2B}$ is $C_{1-4}$alkyl not containing an asymmetric carbon atom, even more preferably $R''_{2B}$, where $R''_{2B}$ is $C_{1-3}$alkyl, and most preferably i-propyl.

In Group IC:

$R_1$ is preferably $R_{1C}$, where $R_{1C}$ is $C_{1-6}$alkyl not containing an asymmetric carbon atom, more preferably $R'_{1C}$, where $R'_{1C}$ is $C_{1-4}$alkyl not containing an asymmetric carbon atom, even more preferably $R''_{1C}$, where $R''_{1C}$ is $C_{1-3}$alkyl, and most preferably i-propyl; and

$R_2$ is preferably $R_{2C}$, where $R_{2C}$ is $C_{1-6}$alkyl not containing an asymmetric carbon atom, more preferably $R'_{2C}$, where $R'_{2C}$ is $C_{1-4}$alkyl not containing an asymmetric carbon atom, even more preferably $R''_{2C}$, where $R''_{2C}$ is $C_{1-3}$alkyl, and most preferably i-propyl.

In each group:

$R_3$ is preferably $R'_3$, where $R'_3$ is $C_{1-4}$alkyl not containing an asymmetric carbon atom or Ring C wherein $R_{11}$ is $R'_{11}$, $R_{12}$ is $R'_{12}$, and $R_{13}$ is $R'_{13}$, more preferably $R''_3$, where $R''_3$ is $C_{1-3}$alkyl or Ring C wherein $R_{11}$ is $R''_{11}$, $R_{12}$ is $R''_{12}$, and $R_{13}$, is hydrogen, even more preferably $R'''_3$, where $R'''_3$ is $C_{1-2}$alkyl, and most preferably methyl.

$R_4$ is preferably $R'_4$, where $R'_4$ is $C_{1-4}$alkyl not containing an asymmetric carbon atom, more preferably $R''_4$, where $R''_4$ is $C_{1-3}$alkyl, even more preferably $R'''_4$, where $R'''_4$ is $C_{1-2}$alkyl, and most preferably methyl.

Each of $R_5$, $R_8$, $R_{11}$ and $R_{14}$ is preferably $R'_5$, $R'_8$, $R'_{11}$ and $R'_{14}$, respectively, where each of $R'_5$, $R'_8$, $R'_{11}$ and $R'_{14}$ is independently hydrogen, $C_{1-3}$alkyl, $C_{1-2}$alkoxy, trifluoromethyl, fluoro or chloro, and more preferably $R''_5$, $R''_8$, $R''_{11}$ and $R''_{14}$, respectively, where each of $R''_5$, $R''_8$, $R''_{11}$ and $R''_{14}$ is independently hydrogen, methyl or fluoro. $R''_5$ and $R''_8$ are most preferably fluoro, especially 4-fluoro, and $R''_{11}$ and $R''_{14}$ are most preferably hydrogen.

Each of $R_6$, $R_9$, $R_{12}$ and $R_{15}$ is preferably $R'_6$, $R'_9$, $R'_{12}$ and $R'_{15}$, respectively, where each of $R'_6$, $R'_9$, $R'_{12}$ and $R'_{15}$ is independently hydrogen, $C_{1-2}$alkyl, fluoro or chloro, more preferably $R''_6$, $R''_9$, $R''_{12}$ and $R''_{15}$, respectively, where each of $R''_6$, $R''_9$, $R''_{12}$ and $R''_{15}$ is independently hydrogen or methyl, and most preferably hydrogen.

Each of $R_7$, $R_{10}$, $R_{13}$ and $R_{16}$ is preferably $R'_7$, $R'_{10}$, $R'_{13}$, and $R'_{16}$, respectively, where each of $R'_7$, $R'_{10}$, $R'_{13}$ and $R'_{16}$ is independently hydrogen or methyl, and most preferably hydrogen.

Preferably, each of Rings A, B, C and D independently bears a maximum of one substituent selected from the group consisting of t-butyl, trifluoromethyl, phenyl, phenoxy and benzyloxy. More preferably, when any two or all three of the substituents on Ring A [$R_5$, ($R'_5$, etc.), $R_6$ ($R'_6$, etc.) and $R_7$ ($R'_7$, etc.)], Ring B [$R_8$, ($R'_8$, etc.), $R_9$ ($R'_9$, etc.) and $R_{10}$ ($R'_{10}$, etc.)], Ring C [$R_{11}$, ($R'_{11}$, etc.), $R_{12}$ ($R'_{12}$, etc.) and $R_{13}$ ($R'_{13}$, etc.)] and Ring D [$R_{14}$, ($R'_{14}$, etc.), $R_{15}$ ($R'_{15}$, etc.) and $R_{16}$ ($R'_{16}$, etc.)] independently are ortho to each other, at least one member of each pair that are ortho to each other is a member of the group consisting of hydrogen, methyl, methoxy, fluoro and chloro. Also more preferably, at least one of the ortho positions of each of Rings A, B, C and D independently has a member of the group consisting of hydrogen, fluoro and methyl.

Each of Rings A and B independently is preferably phenyl, 4-fluorophenyl or 3,5-dimethylphenyl and most preferably 4-fluorophenyl.

Each of Rings C and D is preferably phenyl.

$R_{17}$ is preferably $R'_{17}$, where $R'_{17}$ is hydrogen or methyl, and most preferably hydrogen.

$R_{18}$ is preferably $R'_{18}$, where $R'_{18}$ is hydrogen, $R'_{19}$ or M, more preferably $R''_{18}$, where $R''_{18}$ is hydrogen, $C_{1-3}$alkyl or M, even more preferably $R'''_{18}$, where $R'''_{18}$ is hydrogen, $C_{1-2}$alkyl or M, and most preferably M, particularly M′ and especially sodium.

$R_{19}$ is preferably a physiologically acceptable and hydrolyzable ester group, more preferably $R'_{19}$, where $R'_{19}$ is $C_{1-3}$alkyl, n-butyl, i-butyl, t-butyl or benzyl, even more preferably $R''_{19}$, where $R''_{19}$ is $C_{1-3}$alkyl, and most preferably $R'''_{19}$, where $R'''_{19}$ is $C_{1-2}$alkyl, especially ethyl.

Any $-CH=CH-$, $-CH=CH-CH_2-$ or $-CH_2-CH=CH-$ as X is preferably trans, i.e., (E).

X is preferably X′, where X′ is $-CH_2CH_2-$ or $-CH=CH-$, and most preferably (E)-$CH=CH-$.

Z is preferably a group of Formula a or c wherein $R_{17}$ is $R'_{17}$ (especially hydrogen), and $R_{18}$ is $R'_{18}$ or a group of Formula b wherein $R_{17}$ is $R'_{17}$ (especially hydrogen), more preferably a group of Formula a wherein $R_{17}$ is hydrogen, and $R_{18}$ is $R''_{18}$ or a group of Formula b wherein $R_{17}$ is hydrogen, even more preferably a group of Formula a wherein $R_{17}$ is hydrogen, and $R_{18}$ is $R'''_{18}$ or a group of Formula b wherein $R_{17}$ is hydrogen, and most preferably a group of Formula a wherein $R_{17}$ is hydrogen, and $R_{18}$ is M, preferably M′ and especially sodium.

m is preferably m′, where m′ is 1 or 2, and most preferably 1.

n is preferably n′, where n′ is 2 or 3, and most preferably 2.

M is preferably free from centers of asymmetry and is more preferably M′, i.e., sodium, potassium or ammonium, and most preferably sodium. For simplicity, each formula in which M appears has been written as if M were monovalent and, preferably, it is. However, it may also be divalent or trivalent and, when it is, it balances the charge of two or three carboxy groups, respectively. Thus, Formula I and every other formula containing an M embrace compounds wherein M is divalent or trivalent, i.e., compounds containing two or three carboxylate-containing anions per cation M.

As between otherwise identical compounds of Formula I, those wherein Z is a group of Formula a are generally preferred over those wherein it is a group of Formula b or c, with those wherein Z is a group of

Formula b being preferred over those wherein it is a group of Formula c.

Insofar as the compounds of Groups IAa, IBa and ICa and each of the subgroups thereof are concerned, the erythro isomers are preferred over the threo isomers, erythro and threo referring to the relative positions of the hydroxy groups in the 3- and 5-positions of the group of Formula a.

Insofar as the compounds of Groups IAb, IBb and ICb and each of the subgroups thereof are concerned, the trans lactones are generally preferred over the cis lactones, cis and trans referring to the relative positions of $R_{17}$ and the hydrogen atom in the 6-position of the group of Formula b.

The preferred stereoisomers of the compounds of Formula I having only two centers of asymmetry wherein X is -CH=CH- or $-CH_2-CH=CH-$, and Z is a group of Formula a are the 3R,5S isomers and the racemate of which each is a constituent, i.e., the 3R,5S-3S,5R (erythro) racemates, especially the 3R,5S isomers.

The preferred stereoisomers of the compounds of Formula I having only two centers of asymmetry wherein X is $-(CH_2)_n-$ or $-CH=CH-CH_2-$, and Z is a group of Formula a are the 3R,5R isomers and the racemate of which each is a constituent, i.e., the 3R,5R-3S,5S (erythro) racemates, especially the 3R,5R isomers.

The preferences set forth in the preceding two paragraphs also apply to the compounds of Groups IAa, IBa and ICa having more than two centers of asymmetry and represent the preferred configurations of the indicated positions.

The preferred stereoisomers of the compounds of Formula I wherein X is -CH=CH- or $-CH_2-CH=CH-$, and Z is a group of Formula b are the 4R,6S isomers and the racemate of which each is a constituent, i.e., the 4R,6S-4S,6R (trans lactone) racemates, especially the 4R,6S isomers.

The preferred stereoisomers of the compounds of Formula I wherein X is $-(CH_2)_n-$ or $-CH=CH-CH_2-$, and Z is a group of Formula b are the 4R,6R isomers and the racemate of which each is a constituent, i.e., the 4R,6R-4S,6S (trans actone) racemates, especially the 4R, 6R isomers.

The preferred stereoisomers of the compounds of Formula I having only one center of asymmetry wherein Z is a group of Formula c are the 3R isomers. This preference also applies to the compounds of Groups IAc, IBc and ICc having more than one center of asymmetry and represents the preferred configuration of the indicated position.

Each of the preferences set forth above applies, not only to the compounds of Formula I, but also to the compounds of Groups IA, IB and IC and those of Groups IAa, IAb, IAc, IBa, IBb, IBc, ICa, ICb and ICc as well as to every other subgroup thereof set forth in the specification, e.g., Groups (i) et seq., unless otherwise indicated. When any preference or group contains a variable, the preferred significances of that variable apply to the preference or group in question, unless otherwise indicated.

Preferred groups of compounds of Groups IAa, IAb, IAc, IBa, IBb, IBc, ICa, ICb and ICc include the compounds of the following groups identified by lower case bold Roman numerals between parentheses:

**(i)** of Group IAa wherein $R_1$ is $R_{1A}$, $R_2$ is $R_{2A}$, $R_3$ is $R'_3$, $R_4$ is $R'_4$, $R_{17}$ is $R'_{17}$, $R_{18}$ is $R'_{18}$, and X is X′,

**(ii)** of **(i)** wherein $R_1$ is $R'_{1A}$, $R_2$ is $R'_{2A}$, $R_3$ is $R''_3$, $R_4$ is $R''_4$, $R_{17}$ is hydrogen, $R_{18}$ is $R''_{18}$, and X is (E)-CH=CH-,

**(iii)** of **(ii)** wherein $R_1$ is $R''_{1A}$, and $R_{18}$ is $R'''_{18}$, especially M,

**(iv)** of **(iii)** wherein $R_2$ is $R''_{2A}$, $R_3$ is $R'''_3$, and $R_4$ is $R'''_4$,

**(v)** of **(iv)** wherein $R_{18}$ is M, particularly M′ and especially sodium,

**(vi)** of Group IAb wherein $R_1$ is $R_{1A}$, $R_2$ is $R_{2A}$, $R_3$ is $R'_3$, $R_4$ is $R'_4$, $R_{17}$ is $R'_{17}$, and X is X′,

**(vii)** of **(vi)** wherein $R_1$ is $R'_{1A}$, $R_2$ is $R'_{2A}$, $R_3$ is $R''_3$, $R_4$ is $R''_4$, $R_{17}$ is hydrogen, and X is (E)-CH=CH-,

**(viii)** of **(vii)** wherein $R_1$ is $R''_{1A}$,

**(ix)** of **(viii)** wherein $R_2$ is $R''_{2A}$, $R_3$ is $R'''_3$, and $R_4$ is $R'''_4$,

**(x)**-**(xiv)** of Group IAc which correspond to Groups **(i)**-**(v)**, i.e., wherein the variables are as defined in said groups,

**(xv)** of Group IBa wherein $R_1$ is $R_{1B}$, $R_2$ is $R_{2B}$, $R_3$ is $R'_3$, $R_4$ is $R'_4$, $R_{17}$ is $R'_{17}$, $R_{18}$ is $R'_{18}$, and X is X′,

**(xvi)** of **(xv)** wherein $R_1$ is $R'_{1B}$, $R_2$ is $R'_{2B}$, $R_3$ is $R''_3$, $R_4$ is $R''_4$, $R_{17}$ is hydrogen, $R_{18}$ is $R''_{18}$, and X is (E)-CH=CH-,

**(xvii)** of **(xvi)** wherein $R_2$ is $R''_{2B}$, and $R_{18}$ is $R'''_{18}$, especially M,

**(xviii)** of **(xvii)** wherein $R_1$ is $R''_{1B}$, $R_3$ is $R'''_3$, and $R_4$ is $R'''_4$,

**(xix** of **(xviii)** wherein $R_{18}$ is M, particularly M′ and especially sodium,

**(xx)** of Group IBb wherein $R_1$ is $R_{1B}$, $R_2$ is $R_{2B}$, $R_3$ is $R'_3$, $R_4$ is $R'_4$, $R_{17}$ is $R'_{17}$, and X is X′,

**(xxi)** of **(xx)** wherein $R_1$ is $R'_{1B}$, $R_2$ is $R'_{2B}$, $R_3$ is $R''_3$, $R_4$ is $R''_4$, $R_{17}$ is hydrogen, and X is (E)-CH=CH-,

**(xxii)** of **(xxi)** wherein $R_2$ is $R''_{2B}$,

**(xxiii)** of **(xxii)** wherein $R_1$ is $R''_{1B}$, $R_3$ is $R'''_3$, and $R_4$ is $R'''_4$,

**(xxiv)**-**(xxviii)** of Group IBc which correspond to Groups **(xv)**-**(xix)**, i.e., wherein the variables are as defined in said groups,

**(xxix)** of Group ICa wherein $R_1$ is $R_{1C}$, $R_2$ is $R_{2C}$, $R_3$ is $R'_3$, $R_4$ is $R'_4$, $R_{17}$ is $R'_{17}$, $R_{18}$ is $R'_{18}$, and X is X′,

**(xxx)** of **(xxix)** wherein $R_1$ is $R'_{1C}$, $R_2$ is $R'_{2C}$, $R_3$ is $R''_3$, $R_4$ is $R''_4$, $R_{17}$ is hydrogen, $R_{18}$ is $R''_{18}$, and X is (E)-CH=CH-,

**(xxxi)** of **(xxx)** wherein $R_1$ is $R''_{1C}$, $R_2$ is $R''_{2C}$, and $R_{18}$ is $R'''_{18}$, especially M,

**(xxxii)** of **(xxxi)** wherein $R_3$ is $R'''_3$, and $R_4$ is $R'''_4$,

**(xxxiii)** of **(xxxii)** wherein $R_{18}$ is M, particularly M′ and especially sodium,

**(xxxiv)** of Group ICb wherein $R_1$ is $R_{1C}$, $R_2$ is $R_{2C}$, $R_3$ is $R'_3$, $R_4$ is $R'_4$, $R_{17}$ is $R'_{17}$, and X is X′,

**(xxxv)** of **(xxxiv)** wherein $R_1$ is $R_{1C}$, $R_2$ is $R''_{2C}$, $R_3$ is $R''_3$, $R_4$ is $R''_4$ $R_{17}$ is hydrogen, and X is

(E)-CH=CH-,

**(xxxvi)** of **(xxxv)** wherein $R_1$ is $R''_{1C}$, and $R_2$ is $R''_{2C}$,

**(xxxvii)** of **(xxxvi)** wherein $R_3$ is $R'''_3$, and $R_4$ is $R'''_4$,

**(xxxviii)**-**(xlii)** of Group ICc which correspond to Groups **(xxix)**-**(xxxiii)**, i.e., wherein the variables are as defined in said groups,

**(xliii)**-**(lvii)** of **(i)**-**(v)**, **(xv)**-**(xix)** and **(xxix)**-**(xxxiii)** wherein the hydroxy groups in the 3- and 5-positions of the group of Formula a have the erythro configuration, and

**(lviii)**-**(lxix)** of **(vi)**-**(ix)**, **(xx)**-**(xxiii)** and **(xxxiv)**-**(xxxvii)** wherein $R_{17}$ and the hydrogen atom in the 6-position of the group of Formula b are trans to each other, i.e., the trans lactones.

Groups **(i)**-**(xlii)** embrace each of the possible stereoisomers, racemates and mixtures of diastereoisomers. Groups **(xliii)**-**(lvii)** embrace the 3R,5S and 3S,5R isomers and the 3R,5S-3S,5R racemates of the compounds wherein X is -CH=CH-, and Groups **(xliii)**, **(xlviii)** and **(liii)** also embrace the 3R,5R and 3S,5S isomers and the 3R,5R-3S,5S racemates of the compounds wherein X is $-CH_2CH_2-$. Groups **(lviii)**-**(lxix)** embrace the 4R,6S and 4S,6R isomers and the 4R,6S-4S,6R racemates of the compounds wherein X is -CH=CH-, and Groups **(lviii)**, **(lxii)** and **(lxvi)** also embrace the 4R,6R and 4S,6S isomers and the 4R,6R-4S,6S racemates of the compounds wherein X is $-CH_2CH_2-$.

A particularly preferred group of compounds of the invention is the compounds of Formula Is

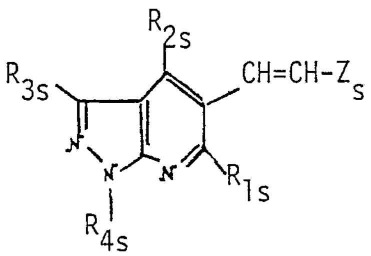

(Is),

wherein

$R_{1s}$ is $C_{1-4}$alkyl not containing an asymmetric carbon atom,

$R_{2s}$ is $C_{1-4}$alkyl not containing an asymmetric carbon atom, $C_{5-7}$cycloalkyl or phenyl optionally monosubstituted by fluoro or chloro,

$R_{3s}$ is $C_{1-4}$alkyl not containing an asymmetric carbon atom or phenyl optionally monosubstituted by fluoro or chloro,

$R_{4s}$ is $C_{1-4}$alkyl not containing an asymmetric carbon atom and

$Z_s$ is $-CH(OH)CH_2CH(OH)CH_2COOR_{18s}$ wherein $R_{18s}$ is hydrogen, $R'_{19}$ or M′ wherein $R'_{19}$ and M′ are as defined above, or is

-a group of formula

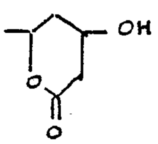

$R_{1s}$ preferably is methyl or isopropyl. $R_{2s}$ conveniently is cyclohexyl or, preferably, 4-fluorophenyl. $R_{3s}$, preferably is methyl or phenyl. $R_{4s}$ preferably is methyl. $Z_s$ preferably is $-CH(OH)CH_2CH(OH)CH_2COOR_{18s}$. That group preferably has the erythro configuration. The carbon atoms carrying the hydroxy substituents in moiety $-CH(OH)CH_2CH(OH)CH_2COOR_{18s}$ preferably are asymmetrically substituted, they preferably have the R configuration for the hydroxy-carrying carbon atom closest to the carboxyl and the S configuration, respectively, for the hydroxy-carrying carbon atom most distant from the carboxyl group. The configuration of the lactone group $Z_s$ preferably is trans, more preferably 4R,6S. $R_{18s}$ preferably is M′. The vinylene moiety in Formula Is is preferably is (E)-CH=CH-.

A compound of the invention may be obtained by a process comprising

a) when X is $-CH_2CH_2-$, $-CH_2CH_2CH_2-$, -CH=CH-, $-CH=CHCH_2-$ or $-CH_2CH=CH-$,

deprotecting a compound of Formula Pa

$PP-X_{aa}-CH(OPro)-CH_2-C(R_{17})(OPro)-CH_2-COOR_{aa}$ (Pa),

wherein

PP is a group of Formula

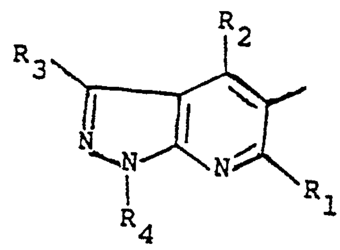

wherein $R_1$ to $R_4$ are as defined above,
$X_{aa}$ is $-CH_2CH_2-$, $-CH_2CH_2CH_2-$, $-CH=CH-$, $-CH=CHCH_2-$or $-CH_2CH=CH-$,
$R_{17}$ is as defined above,
Pro is a protecting group and
$R_{aa}$ is a radical forming an ester,

b) when $R_{18}$ is hydrogen or M,
hydrolysing a compound of Formula I in the form of an ester or lactone,

c) when X is $-(CH_2)_n-$ or (E)-CH=CH- and Z is a group of Formula a wherein $R_{17}$ is hydrogen, reducing a compound of Formula Pc
PP-$X_1$-CH(OH)-$CH_2$-C(=O)-$CH_2$-$COOR_{aa}$ (Pc),
wherein
PP and $R_{aa}$ are as defined above and
$X_1$ is $-(CH_2)_n-$ or (E)-CH=CH-,

d) when X is $-(CH_2)_n-$ or (E)-CH=CH- and Z is a group of Formula a wherein $R_{17}$ is $C_{1-3}$ alkyl, hydrolysing a compound of Formula Pd
PP-$X_1$-CH($OCOR_{20}$)-$CH_2$-C($R_{17a}$)(OH)-$CH_2$-$COOR_{aa}$ (Pd),
wherein
PP, $X_1$ and $R_{aa}$ are as defined above,
$R_{17a}$ is $C_{1-3}$alkyl and
$R_{20}$ is part of an ester forming group,

e) when Z is a group of Formula c, oxidising a corresponding compound of Formula I wherein Z is a group of Formula a, or

f) esterifying or lactonising a compound of Formula I in free acid form,
and when a free carboxyl group is present, recovering the compound obtained in free acid form or in the form of a salt.

The above process variants may be effected in conventional manner, e.g. as described in EP 200736, EP 216785 and EP 235164.

Unless otherwise stated reactions are performed in a manner conventional for the type of reaction involved. Molar ratios and reaction times are as a rule conventional and non-critical and are chosen according to principles well established in the art on the basis of reactions and conditions employed.

Solvents, alone or as mixtures, are generally chosen which remain inert and liquid during the reaction in question.

Examples of inert atmospheres are usually nitrogen or a noble gas, dry nitrogen and argon being preferred. Most reactions are carried out under such for convenience.

Process variant a) is particularly suited for compounds in ester form. Process variant c) is particularly suited for compounds wherein X is $-(CH_2)_n-$ or (E)-CH=CH- and in ester form. Process variant d) is particularly suited for compounds wherein X is $-(CH_2)_n-$ or (E)-CH=CH- and in salt form.

$R_{aa}$ preferably is $C_{1-3}$alkyl, n-butyl, i-butyl, t-butyl or benzyl, more preferably $C_{1-3}$alkyl and especially $C_{1-2}$alkyl. $R_{20}$ preferably is $C_{1-3}$alkyl, more preferably n-$C_{1-3}$alkyl, especially $C_{1-2}$alkyl.

Deprotection according to process variant a) is carried out in conventional manner e.g. by cleavage under mild conditions such as employing e.g. for removal of a diphenyl-t-butylsilyl group a fluoride reagent, e.g. tetra-n-butylammonium fluoride in an anhydrous inert organic medium, preferably tetrahydrofuran, containing glacial acetic acid, at temperatures of approximately 20° to approximately 65°C, especially 20° to 25°C. Preferably 1-6 moles of fluoride per mole protecting group are used with 0.8-2 moles, preferably 1 to 1.5 moles of glacial acetic acid to each mole of fluoride.

Examples of protecting groups Pro are diphenyl-t-butylsilyl, tri-isopropylsilyl or dimethyl-t-butylsilyl. Especially preferred are trisubstituted silyl radicals, in particular diphenyl-t-butylsilyl (Pro′).

Hydrolysis according to process variants b) and d) is carried out in a manner conventional for such reactions, e.g. employing an inorganic hydroxide such as NaOH or KOH with, if desired, subsequent acidification to give the free acid form. Suitable solvents are mixtures of water and water miscible solvents such as lower alkanols, e.g. methanol or ethanol, and reaction conveniently takes place at temperatures of from about 0°C to reflux, preferably 0° to 75°C, e.g. 20° to 70°C. If it is desired to recover the compound in a salt form corresponding to the cation of the hydroxide employed then slightly less than equivalent amounts of

the latter may be employed. In process variant d) $R_{aa}$ will conveniently be the same as $R_{20}$, e.g. $C_{1-3}$alkyl, more preferably n-$C_{1-3}$alkyl, especially $C_{1-2}$alkyl, but $R_{aa}$ and $R_{20}$ may also be different.

Reduction according to process variant c) is preferably carried out using a mild reducing agent such as sodium borohydride or a complex of t-butylamine and borane in an inert organic solvent such as a lower alkanol, preferably ethanol, conveniently at a temperature of from about -10° to about 30°C, under an inert atmosphere.

Use of an optically pure starting material will lead to only two optical isomers (diastereoisomers) of the resulting end product. However, if stereospecificity is desired it is preferred to utilize a stereoselective reduction in order to maximize production of a mixture of the erythro stereoisomers (racemate), of which the preferred steroisomer (as set forth above) is a constituent. Stereoselective reduction is preferably carried out in three steps. For example in a first step, the ketoester of Formula Pc is treated with a tri(primary or secondary $C_{2-4}$alkyl)borane, preferably triethylborane or tri-n-butylborane, and optionally air to form a complex. The reaction temperature is suitably from about 0° to about 50°C, preferably 0° to 25°C. The first step is carried out in an anhydrous inert organic solvent, preferably an ether solvent such as tetrahydrofuran, diethyl ether, 1,2-dimethoxyethane or 1,2-diethoxyethane, with tetrahydrofuran being the most preferred solvent, especially in a 3-4 : 1 mixture with methanol when pure erythro product is desired. In a second step, the complex is reduced with sodium borohydride, preferably in the same solvent as utilized for the first step, at from about -100° to about -40°C, preferably from -90° to -70°C. In a third step, the product of the second step is, for example, treated with, preferably, anhydrous methanol at from about 20° to about 60°C, preferably from 20° to 25°C. The amount of methanol is not critical. However, a large excesss, e.g. 50-100 moles per mole of ketoester of Formula Pc is typically utilized.

Oxidation according to process variant e) can be carried out in conventional manner, e.g. when X is $-CH=CH-$ or $-CH_2CH=CH-$ using activated $MnO_2$ at from about 20° to about 80°C, preferably 40° to 80°C in an inert organic solvent such as an ether solvent, e.g. $(C_2H_5)O$, 1,2-diethoxyethane, 1,2-dimethoxyethane, tetrahydrofuran and mixtures thereof, or e.g. when X is $-(CH_2)_n-$ or $-CH=CH-CH_2-$ using Swerns reagent (oxalyl chloride + dimethylsulfoxide) with triethylamine in e.g. $CH_2Cl_2$ at temperatures of from about -60° to about -40°C, preferably -50°C.

Esterification according to process variant f) is also conventional, employing e.g. a large excess of a compound $R_{19}OH$, wherein $R_{19}$ is as defined above, at e.g. from about 20° to about 40°C, in the presence of a catalytic amound of an acid such as p-toluenesulfonic acid. Direct esterification is particularly suited when Z is a group of Formula c. Preferably, however, esterification takes place by first forming the corresponding lactone and reacting this with $M_2^+$ $-OR_{13}$ ($M_2^+=$ $Na^+$ or $K^+$) at from about 0° to about 70°C, preferably 20° to 25°C, in an inert organic solvent, e.g. an ether such as tetrahydrofuran or an alcohol of Formula $R_{19}OH$ if a liquid.

Lactonisation according to process variant f) is also carried out in conventional manner, e.g. by heating the corresponding acid in an anhydrous inert organic solvent, e.g. a hydrocarbon such as benzene, toluene or a xylene or mixtures thereof, preferably at temperatures of from about 75° to reflux, although more preferably not above 150°C. Preferably, however, a lactonisation agent, e.g. a carbodiimide, preferably a water-soluble carbodiimide such as 1-cyclohexyl-3-(2-morpholinoethyl)carbodiimide metho-p-toluenesulfonate, in an anhydrous inert organic solvent, e.g. a halogenated lower alkane, preferably methylene chloride is employed. Reaction temperatures then lie typically between about 10° and about 35°C, especially 20° to 25°C.

As is evident to those in the art, a racemic threo 3,5-dihydroxycarboxylic acid yields a racemic cis lactone (two stereoisomers) and a racemic erythro 3,5-dihydroxycarboxylic acid yields a racemic trans lactone (two stereoisomers). Likewise, if a single enantiomer of the 3,5-dihydroxycarboxylic acid is utilized, a single enantiomer of the lactone is obtained. For example, lactonisation of a 3R,5S erythro dihydroxycarboxylic acid yields a 4R,6S lactone.

It will readily be appreciated that the various forms of the compound of Formula I may be interconverted as indicated in process variants b) and f) above, whereby lactonisation may only take place when Z is a group of Formula a.

In the same way compounds obtained according to process variants a) and c) to f) above may be as appropriate hydrolysed to free acid forms and free acid forms may be esterified or lactonised to produce a desired end product. The invention thus also comprises a process for preparing a compound of Formula I which comprises hydrolysing a compound of Formula I in ester or lactone form or esterifying or lactonising a compound of Formula I in free acid form and when a free carboxyl group is present, recovering the compound obtained in free acid form or in the form of a salt.

The following Reaction Schemes further illustrate the above process variants. The required starting materials may be prepared for example as is also illustrated in the following Reaction Schemes, or in the Examples hereinafter. Further suitable reaction conditions are disclosed e.g. in EP 200736, EP 216785 and EP 235164, including the Examples thereof.

Thus process variant a) (deprotection) is illustrated in Reaction Scheme II as reactions H and J leading to compounds XXIII and XXV, together with the preparation of the corresponding starting materials of Formula Pa as compounds XXII and XXIV.

Process variant b) (hydrolysis) is illustrated in Reaction Scheme III as reactions K, P and O leading to compounds XXXII and XXXVIII, and in Reaction Scheme IV as reaction S leading to compounds LXIX.

Process variant c) (reduction) is illustrated in Reaction Scheme I as reaction B leading to compounds

IX, together with the preparation of the corresponding starting materials of Formula Pc as compounds VIII.

Process variant d) (hydrolysis) is illustrated in Reaction Scheme I as reaction F leading to compounds XVIII, together with the preparation of the corresponding starting materials of Formula Pd as compounds XVI.

Process variant e) (oxidation) is illustrated in Reaction Scheme IV as reaction R leading to compounds LXVIII.

Process variant f) (esterification or lactonisation) is illustrated in Reaction Scheme III as reactions Q (esterification) and N (lactonisation) leading to compounds XXXVIII and XXXVI, respectively, and in Reaction Scheme IV as reaction V (esterification) leading to compounds LXXII.

The recovery in free acid or in salt form is variously illustrated in Reaction Schemes I (reaction F), III (reactions L and M) and IV (reactions T and U).

**Reaction Scheme I**

The compounds of Formula I wherein X is $-(CH_2)_n-$ or (E)-CH=CH-, and Z is a group of Formula a wherein $R_{17}$ is hydrogen and $R_{18}$ is $R'_{19}$, or $R_{17}$ is $R_{17a}$ and $R_{18}$ is $M_2^+$ may be synthesized by the following series of reactions:

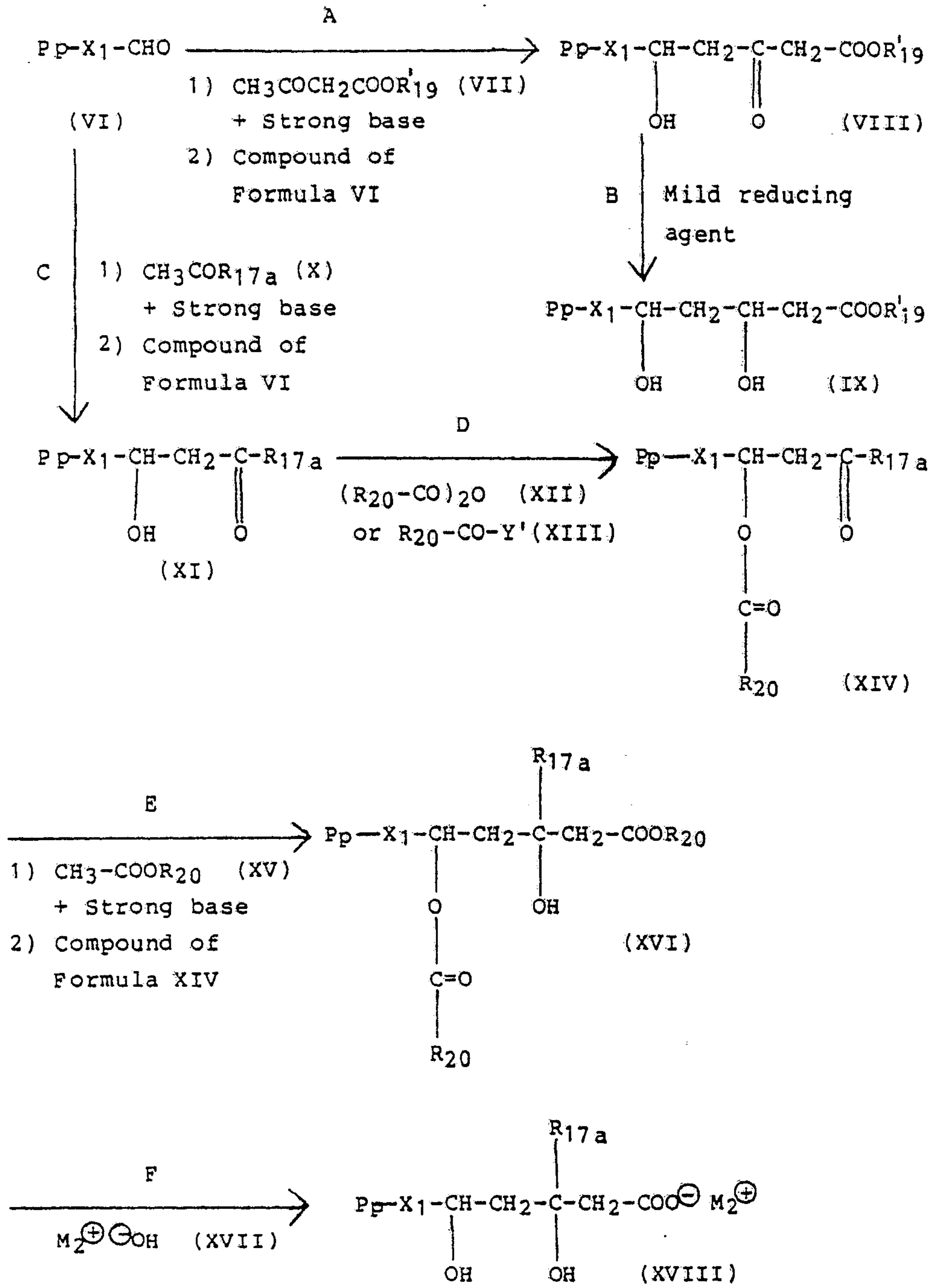


(VI) (VIII) (IX) (XI) (XIV) (XVI) (XVIII)

**Reaction Scheme II**

The compounds of Formula I wherein X is $-CH_2-CH_2-$, $-CH_2CH_2CH_2-$, $-CH=CH-$, $-CH=CH-CH_2-$ or $-CH_2-CH=CH-$, and Z is a group of Formula a wherein $R_{18}$ is $R'_{19}$ may be synthesized by the following series of reactions:

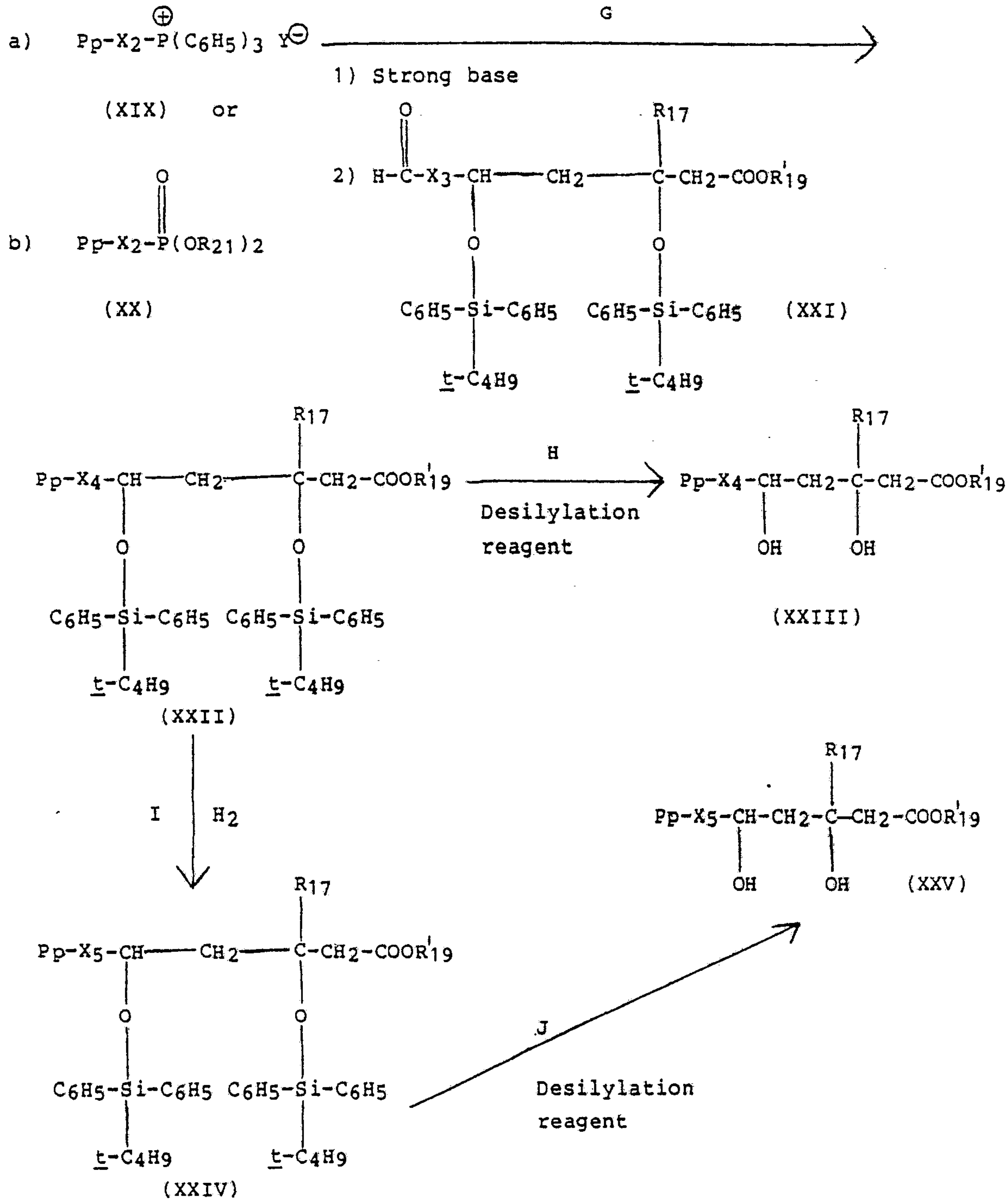


a) (XIX) or b) (XX)

(XXI)

(XXII)

(XXIII)

(XXIV)

(XXV)

**Reaction Scheme III**

The compounds of Formula I wherein Z is a group of Formula a wherein $R_{18}$ is $R'_{19}$ may be converted into the corresponding compounds of Formula I wherein Z has a different significance of Formula a or b by the following series of reactions:

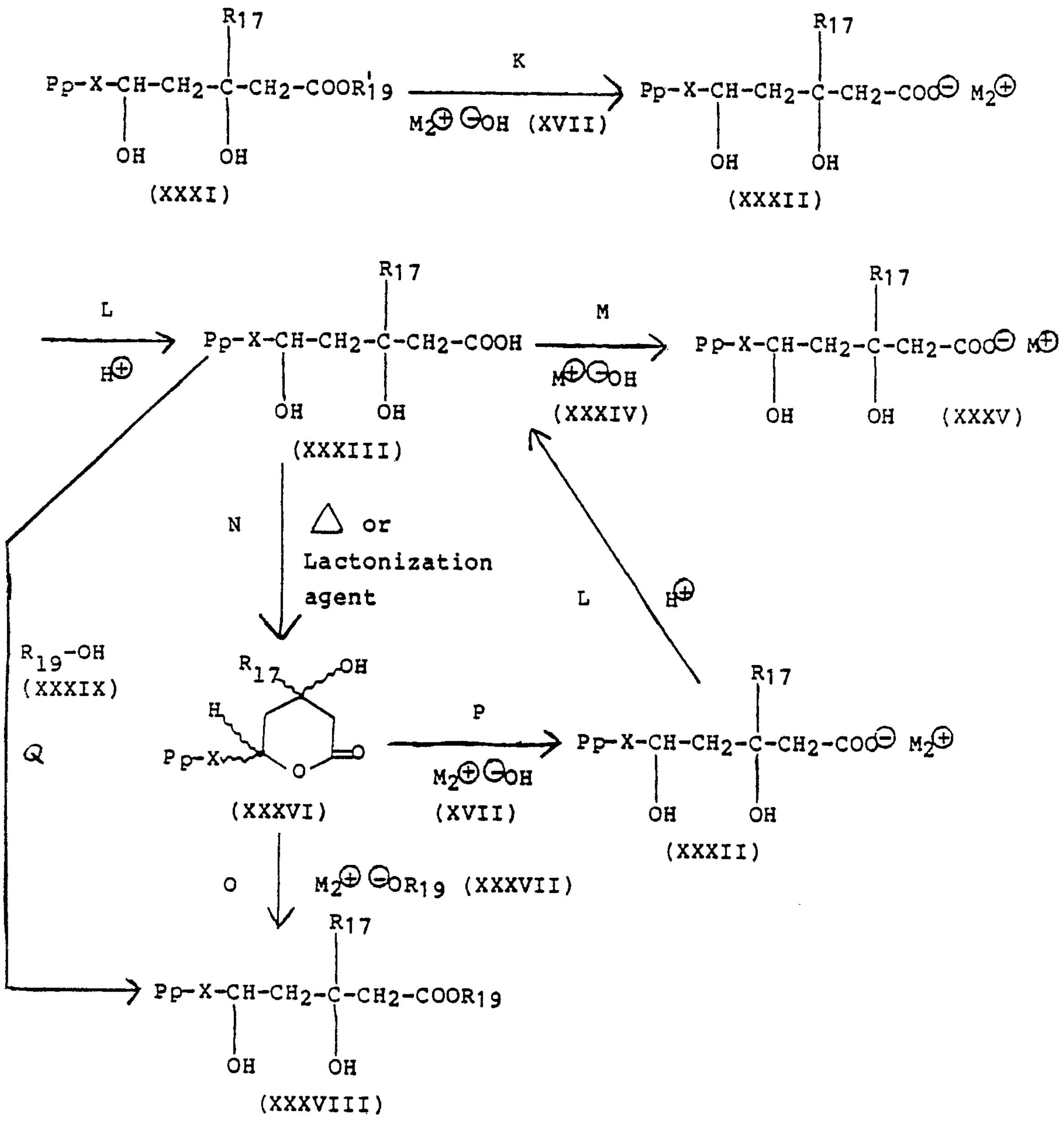


**Reaction Scheme IV**

The compounds of Formula I wherein Z is a group of Formula c may be synthesized by the following series of reactions:

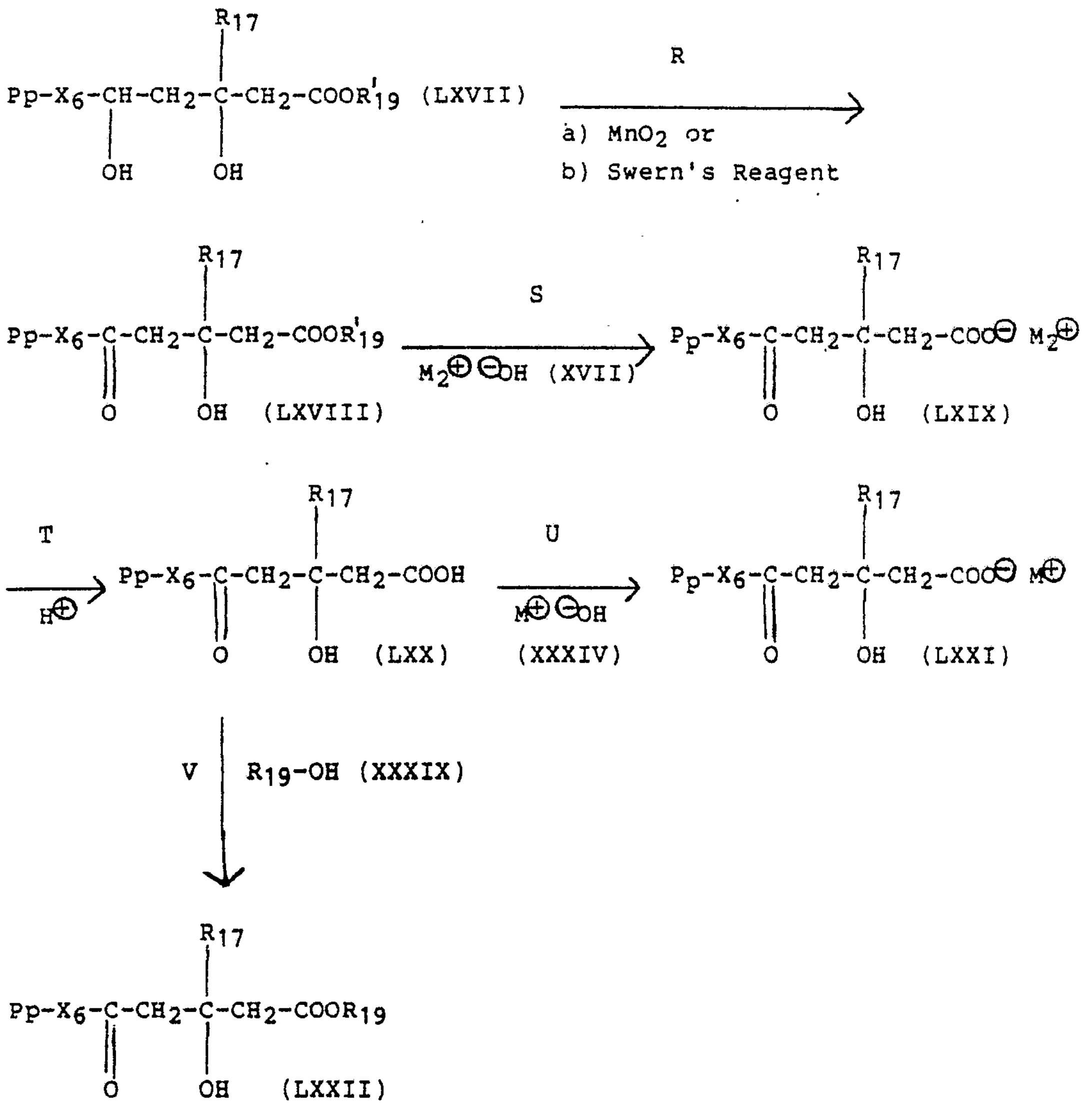


**Reaction Scheme V**

The compounds of Formula VI may be synthesized by the following series of reactions:

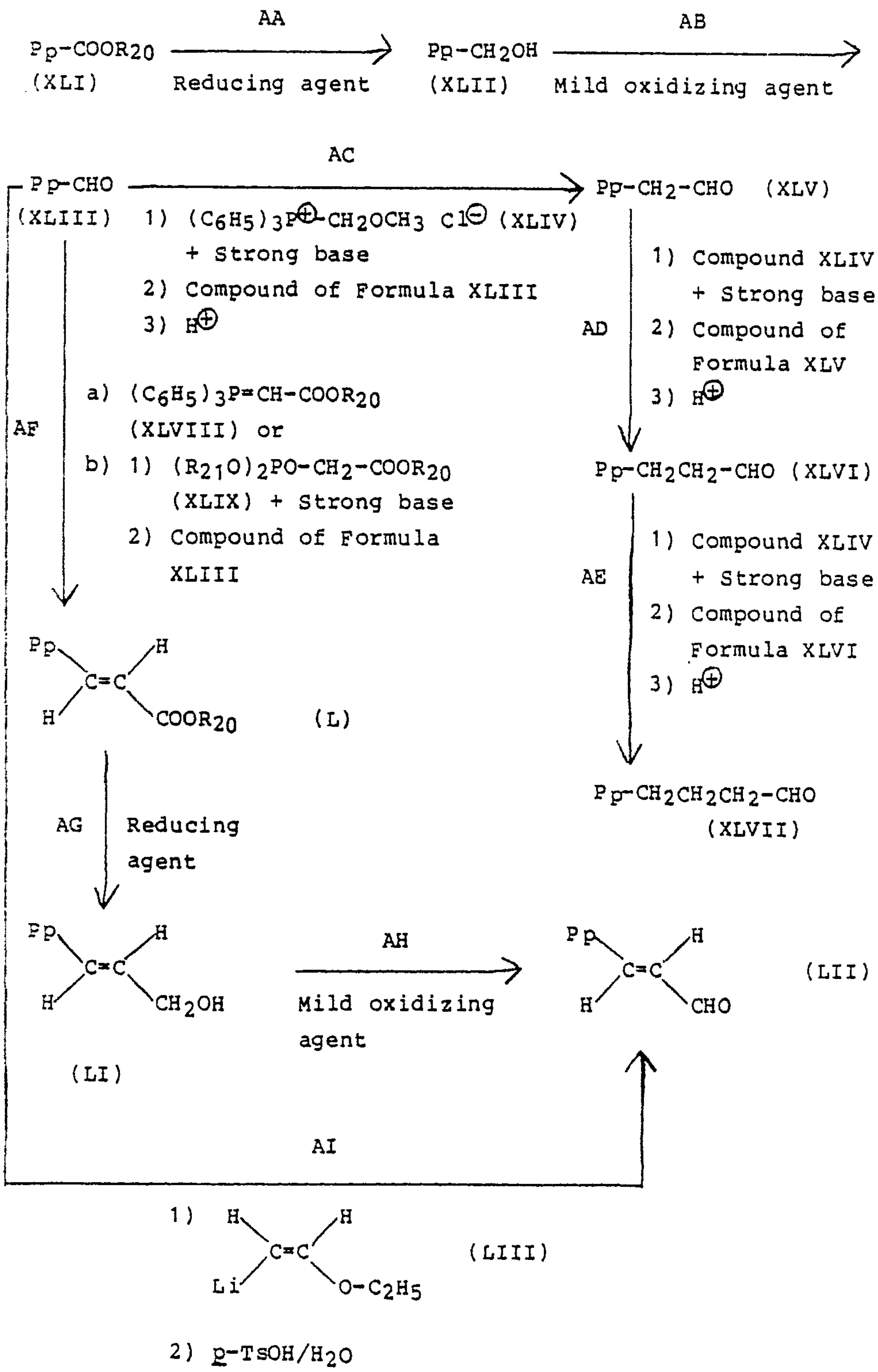
AA
(XLI)
Reducing agent
(XLII)
AB
Mild oxidizing agent
AC
(XLIII)
1) $(C_6H_5)_3P^{\oplus}$-$CH_2OCH_3$ $Cl^{\ominus}$ (XLIV)
+ Strong base
2) Compound of Formula XLIII
3) $H^{\oplus}$
(XLV)
AD
1) Compound XLIV
+ Strong base
2) Compound of
Formula XLV
3) $H^{\oplus}$
(XLVI)
AE
1) Compound XLIV
+ Strong base
2) Compound of
Formula XLVI
3) $H^{\oplus}$
(XLVII)
AF
a) $(C_6H_5)_3P$=CH-$COOR_{20}$
(XLVIII) or
b) 1) $(R_{21}O)_2PO$-$CH_2$-$COOR_{20}$
(XLIX) + Strong base
2) Compound of Formula
XLIII
(L)
AG
Reducing
agent
(LI)
AH
Mild oxidizing
agent
(LII)
AI
1)
(LIII)
2) p-TsOH/$H_2O$

**Reaction Scheme VI**

The compounds of Formulae XIX, XX and XLI may be synthesized by the following series of reactions:

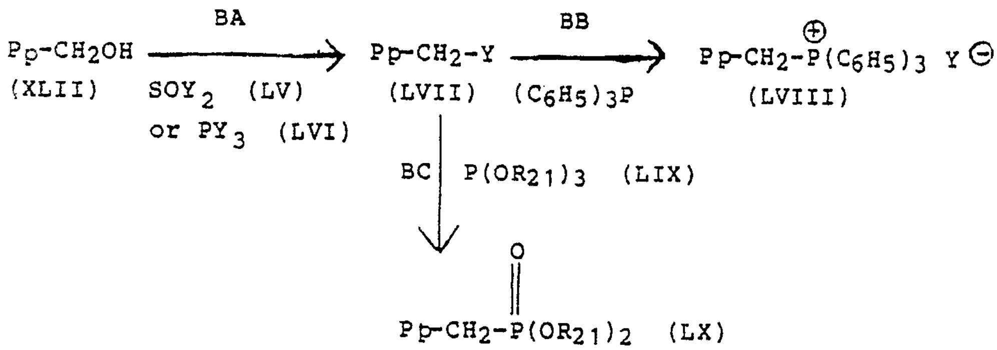
BA
BB
(XLII)
$SOY_2$ (LV)
or $PY_3$ (LVI)
(LVII)
$(C_6H_5)_3P$
(LVIII)
BC
$P(OR_{21})_3$ (LIX)
(LX)

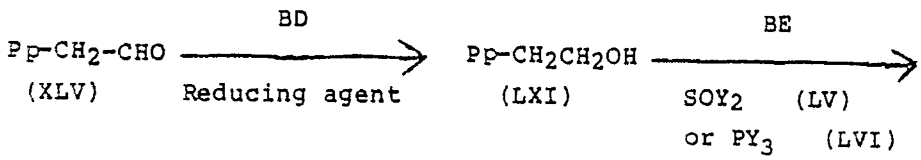
BD
(XLV)
Reducing agent
(LXI)
BE
$SOY_2$ (LV)
or $PY_3$ (LVI)

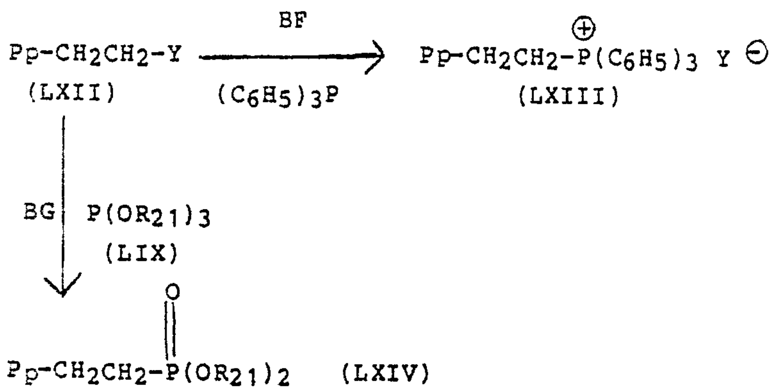
BF
(LXII)
$(C_6H_5)_3P$
(LXIII)
BG
$P(OR_{21})_3$
(LIX)
(LXIV)

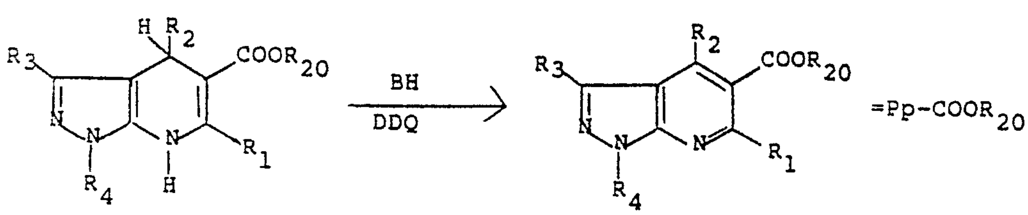
BH
DDQ
=Pp-COOR20

(LXV) (XLI)

In the preceding Reaction Schemes,
Pp is a group PP as defined above,
$R_{17a}$ is $C_{1-3}$alkyl,
each $R_{20}$ is independently $C_{1-3}$alkyl, preferably n-$C_{1-3}$alkyl, and most preferably $C_{1-2}$alkyl,
each $R_{21}$ is independently $C_{1-2}$alkyl, the two $C_{1-2}$alkyl groups preferably being the same,
$X_1$ is $-(CH_2)_n-$ or (E)-CH=CH-, especially (E)-CH=CH-, wherein n is 1, 2 or 3,
$X_2$ is $-CH_2-$ or $-CH_2CH_2-$,
$X_3$ is a direct bond or $-CH_2-$,
$X_4$ is -CH=CH-, -CH=CH-$CH_2$- or -$CH_2$-CH=CH-, preferably (E)-CH=CH-, (E)-CH=CH-$CH_2$- or (E)-$CH_2$-CH=CH- and especially (E)-CH=CH-,
$X_5$ is $-CH_2CH_2-$ or $-CH_2CH_2CH_2-$, especially $-CH_2CH_2-$,
$X_6$ is $-(CH_2)_n-$, -CH=CH-, -$CH_2$-CH=CH- or -CH=CH-$CH_2$- when $R_{17}$ is $C_{1-3}$alkyl and is -CH=CH- or -$CH_2$-CH=CH- when $R_{17}$ is hydrogen, wherein n is 1, 2 or 3,
Y is chloro or bromo,
$Y^-$ is chloride or bromide,
$M_2^+$ is sodium or potassium, and
each of the other variables is as defined above.

As is evident to those in the art, each of the compounds of Formulae I wherein Z is a group of Formula c (including those of Formulae LXVIII-LXXII), VIII, XI, XIV and LXV and has a single center of asymmetry and, therefore, may be resolved into two optically active isomers. When a compound of Formula VIII or XIV is converted into a compound of Formula IX or XVI, respectively, an additional center of asymmetry is generated. Consequently, when a racemic compound of Formula VIII or XIV is utilized, four stereoisomers (two pairs of diastereoisomers) of the resulting compound of Formula IX or XVI are formed, whereas when an optically pure compound of Formula VIII or XIV is utilized, two diastereoisomers of the compound of Formula IX or XVI are formed.

The compounds of Formulae I wherein Z is a group of Formula a or b (including those of Formulae IX, XVIII, XXIII, XXV, etc.), XVI, XXI, XXII and XXIV have two centers of asymmetry and, therefore, may exist in four stereoisomeric forms. Except where the compound is formed from an optically pure precursor already having both chiral carbon atoms or where the reaction involves the use of a stereospecific reagent that gives an optically pure product, the compound is obtained as a mixture of two (if formed from an optically pure compound having one center of asymmetry) or four (if formed from a racemic compound having one center of asymmetry) stereoisomers.

The center of asymmetry of each compound of Formula LXV may be ignored since it is destroyed in the following reaction (Reaction BH).

The obtained mixtures of stereoisomers may be separated by conventional means. For example, diastereoisomers may be separated by fractional crystallization, column chromatography, preparative thin layer chromatography and HPLC. Each mixture of four stereoisomers of a compound of Formula XXXVI may, for example, be separated by HPLC into its cis and trans (lactone) components, each of which is a racemate that may be resolved into two optically active enantiomers.

Techniques for separating a racemate into its two optically active enantiomers are known. For example, a racemic compound having a carboxylic acid group may be reacted with an optically pure organic base having at least one center of asymmetry to form a mixture of diastereoisomeric salts that may be separated by fractional crystallization, column chromatography, etc. or it may be reacted with an optically pure alcohol having at least one center of asymmetry to form a mixture of diastereoisomeric esters which may be separated by conventional techniques such as those set forth above or below. Likewise, a racemic compound having a carboxylic acid, acyl halide, ester or lactone group may be reacted with an optically pure organic base, i.e., an amine, to form a mixture of diastereoisomeric amides that may be separated by conventional means, e.g., fractional crystallization, column chromatography and/or HPLC. For example, a racemic lactone of Formula XXXVI may be reacted with an excess of R-(+)-α-methylbenzylamine (or the corresponding S-(-) compound) to form a mixture of two diastereoisomeric α-methylbenzylamides which may be separated by, for example, column chromatography on a silica gel column and/or by HPLC using a Partisil column. Often it is desirable to utilize both techniques, i.e., to partially separate the diastereoisomers by column chromatography and to purify each fraction by HPLC. Typically, the α-methylbenzylamides are synthesized by reacting the racemic lactone with a large molar excess of the amine at 20°-25°C for 16-24 hours. The reaction is run neat, with the excess amine serving as the solvent. After the reaction, the excess amine is removed by vacuum distillation at 25°-35°C. After separation, each chiral amide may be hydrolyzed to the corresponding, for example, sodium, salt by, for example, refluxing with 1.5-3, preferably 2-2.2, equivalents of a base such as sodium hydroxide for 5-25 hours in a mixture of water and ethanol. The resulting salts may be converted to the corresponding free acids, esters, lactones and other salts by conventional means such as the reactions set forth in Reaction Scheme III. On the other hand, a racemic compound having a hydroxy group may be esterified with an optically pure carboxylic acid having at least one center of asymmetry to form a mixture of diastereoisomeric esters or it may be reacted with an optically pure trisubstituted silyl halide, e.g., (-)-α-naphthylphenylmethylchlorosilane (Sommer et al., J. Am. Chem. Soc. 80, 3271 [1958]), to form a mixture of two diastereoisomeric silyloxy compounds, which mixture may be separated by conventional techniques. For example, diastereoisomeric (-)-α-naphthylphenylmethylsilyl derivatives of a lactone type end product of Formula I or XXXVI may be

separated on a silica column having covalently bound L-phenyl-glycine. After separation, the optically pure salts, amides, esters or silyloxy compounds are reconverted to the corresponding carboxy group- or hydroxy group-containing compounds with retention of optical purity. For example, the process conditions disclosed for Reactions H and J may be utilized to cleave (-)-α-naphthylphenylmethylsilyl and other silyl groups.

The conditions given hereinabove are largely conventional for such reactions and can be varied in conventional manner according to the particular intermediates/end products. This applies e.g. to molar ratios, temperature, reaction times and the like which are chosen according to principles well established in the art on the basis of reactants and conditions employed.

Intermediates the production of which is not described above are either known or may be prepared according to or analogously to known methods, e.g. as described in EP 200736, EP 216785 and EP 235163, including the Examples thereof.

Reaction products, both intermediates and final, can be isolated (e.g. from compound mixtures or reaction mixtures) and purified in conventional manner, whereby intermediates can, where appropriate, be employed directly in a subsequent reaction.

Mixtures of isomers (cis, trans and optical) can be separated by conventional means at whatever stage of synthesis is appropriate. Such methods include re-crystallisation chromatography, formation of esters with optically pure acids and alcohols or of amides and salts with subsequent reconversion under retention of optical purity.

Salts may be prepared in conventional manner from free acids, lactones and esters and vice-versa. In some cases ion-exchange may be used for salt formation. Whilst all salts are covered by the invention, pharmaceutically acceptable salts, especially sodium, potassium and ammonium, particularly sodium salts are preferred.

The various forms of the compounds of Formula I are by virtue of their interconvertability useful as intermediates in addition to the uses set out below.

Also within the scope of this invention are the intermediates of Formulae VIII, XI, XIV, XVI, XIX, XX, XXII, XXIV, XLI-XLIII, XLV-XLVII, L-LII, LVII, LXI and LXII.

The compounds of Formula I in free acid form, or in the form of a physiologically acceptable ester or a δ-lactone thereof, or in pharmaceutically acceptable salt form, hereinafter referred to as "the compounds", exhibit pharmacological activity and are, therefore, indicated for use as pharmaceuticals.

In particular, the compounds are competitive inhibitors of 3-hydroxy-3-methylglutaryl coenzyme A (HMG-CoA) reductase, the rate limiting enzyme in cholesterol biosynthesis, and, therefore, they are inhibitors of cholesterol biosynthesis.

The biological activity of the compounds may be demonstrated in the following two tests:

Test A. In Vitro Microsomal Assay of HMG-CoA

Reductase Inhibition:

This test is carried out precisely as described on page 30 of WO 84/2131. The concentration of the test substance in the assay system is 0.001-2,000 μmolar. The obtained $IC_{50}$ is the concentration of the test substance in the assay system observed or calculated to produce a 50% inhibition of HMG-CoA reductase activity.

Test B. In Vivo Cholesterol Biosynthesis

Inhibition Test:

This test is carried out precisely as described on page 33 of WO 84/2131. In this test the rats are orally administered the test substance at a dose of 0.1-200 mg/kg body weight. The obtained $ED_{50}$ is the dose of the test substance observed or calculated to produce a 50% inhibition of 3β-hydroxysterol synthesis.

The compounds are, therefore, indicated for use in lowering the blood cholesterol level and, therefore, as hypolipoproteinemic and anti-atherosclerotic agents.

The compounds may be administered by any conventional route, in particular enterally, preferably orally, e.g., in the form of tablets or capsules, or parenterally, e.g., in the form of sterile injectable solutions or suspensions.

An indicated suitable daily dosage for use in the treatment of hyperlipoproteinemia and atherosclerosis is from about 2.5 mg to about 2 g, suitably administered one to four times daily or in controlled release form. A typical dosage unit for oral administration may contain from about 0.25 mg to about 1 g, especially from about 0.6 mg to about 500 mg.

The compounds may be administered in similar manner as known compounds suggested for use in such indication, e.g. Compactin or Mevinolin. The suitable daily dosage for a particular compound will depend on a number of factors such as its relative potency of activity.

The compound of Example 2 is the preferred compound. It has, for example, been determined that in Test A this compound has an $IC_{50}$ of 0.01 μmolar as compared to an $IC_{50}$ of 1.01 μmolar for Compactin and an $IC_{50}$ of 0.14 μmolar for Mevinolin and, in Test B, this compound has an $ED_{50}$ of 0.05 mg/kg as compared to an $ED_{50}$ of 3.5 mg/kg for Compactin and an $ED_{50}$ of 0.38 mg/kg for Mevinolin.

It is, therefore, indicated that the compounds may be administered at dosages significantly lower than those

conventionally employed with Compactin and Mevinolin (e.g., 20-80 mg/day orally of Mevinolin).

The invention thus also concerns a method of treating hyperlipoproteinemia or atherosclerosis by administration of a compound of Formula I in free acid form or in the form of a physiologically acceptable ester or a δ-lactone thereof or in pharmaceutically acceptable salt form, as well as such compounds for use as pharmaceuticals, e.g. as hypolipoproteinemic and anti-atherosclerotic agents.

The compounds may be administered alone or in admixture with a pharmaceutically acceptable diluent or carrier and, optionally, other excipients, and administered orally in such forms as tablets or capsules, or parenterally in such forms as injectable solutions or suspensions.

The preferred pharmaceutical compositions from the standpoint of ease of preparation and administration are solid compositions, particularly tablets and hard-filled or liquid-filled capsules.

Such compositions also form part of the invention. They may be manufactured by mixing in conventional manner with a pharmaceutically acceptable carrier or diluent.

The following Examples illustrate the invention.

## Example 1

**1,1-Dimethylethyl (3R,5S)-(E)-3,5-dihydroxy-7-[1′,3′-dimethyl-4′-(4″-fluorophenyl)-6′-(1″-methylethyl)-1H-pyrazolo[3,4-b]pyrid-5′-yl]hept-6-enoate**

[deprotection; process variant a); reaction H]

A solution of 32.7 g of 1,1-Dimethylethyl (3R,5S)-(E)-7-[1′,3′-dimethyl-4′-(4″-fluorophenyl)-6′-(1″-methylethyl)-1H-pyrazolo[3,4-b]pyrid-5′-yl]-3,5-di[(1′,1′-dimethylethyl)diphenylsilyloxy]hept-6-enoate (product of Step 9 [see below]) in 200 ml of acetonitrile is added rapidly dropwise to a mixture of 107 g of tetra-n-butylammonium fluoride·trihydrate, 500 ml of acetonitrile and 20.4 g of glacial acetic acid stirred at 45°-50°C, and the reaction mixture is stirred at 60°-65°C for 48 hours, the reaction mixture being stirred under argon throughout. The reaction mixture is poured into a mixture of 150 ml of saturated sodium chloride solution, 200 ml of saturated sodium carbonate solution and 1.35 l of water (the pH of the mixture should be ~7.5-8.5 after the addition), and the mixture is extracted three times with diethyl ether. The diethyl ether extracts are combined, washed three times with 500 ml portions of water, dried over anhydrous magnesium sulfate, filtered and evaporated at reduced pressure to an orange oil. The obtained oil is flash chromatographed on 230-400 mesh ASTM silica gel utilizing 7:3 mixed hexanes/ethyl acetate as the eluant. The fractions containing the product (as indicated by thin layer chromatography) are combined and evaporated at reduced pressure to obtain the product as a resin.

N.M.R. ($CDCl_3$): 1.33-1.37 (d, 6H), 1.51 (s, 9H), 1.97 (s, 3H), 2.32-2.40 (m, 2H), 3.39-3.53 (m, 1H), 4.12 (s, 3H), 4.31-4.45 (m, 1H), 5.26-5.38 (dd, 1H), 6.50-6.59 (d, 1H), 7.08-7.31 (m, 4H)

The starting material is obtained as follows:

### Step 1

5-Amino-1,3-dimethylpyrazole

A mixture of 250 g of 3-aminocroto-nitrile, 152.5 g of methylhydrazine and 1 l of absolute ethanol is refluxed for 8 hours (until the evolution of ammonia ceases) and cooled to 20°-25°C. The ethanol is evaporated at reduced pressure, and the residual oil is triturated with 2300 ml of benzene. The resulting yellow-orange solid product is collected by filtration and vacuum dried at 45°C to obtain the product (M.P. 74°-76°C).

### Step 2

Ethyl (Z)-2-[(4′-fluorophenyl)methylene]-4-methyl-3-oxopentanoate

A mixture of 981 g of ethyl 4-methyl-3-oxopentanoate, 655 g of 4-fluoro-benzaldehyde, 80 ml of glacial acetic acid, 80 ml of dry piperidine and 2 l of benzene is refluxed for 7-8 hours to collect 105 ml of water, cooled to 20°-25°C and poured into 4 l of cold water. 950 ml of diethyl ether is added, the mixture is stirred for 5 minutes, the organic layer is separated, the aqueous layer is extracted with 950 ml of diethyl ether, and the organic phases are combined, washed successively with 1 l of 2N sodium hydroxide solution, 1 l of saturated sodium bisulfite solution and then water until neutral to pH paper, dried over anhydrous magnesium sulfate, filtered and evaporated at reduced pressure to obtain an orange-yellow oil which is fractionally vacuum distilled to obtain the product as an oil (B.P. 135°-145°C/0.7-1.4 mm Hg; 133°-138°C/0.3-0.4 mm Hg).

### Step 3

Ethyl 3-(5′-amino-1′,3′-dimethyl-1H-pyrazol-4′-yl)-3-(4′-fluorophenyl)-2-(2′-methyl-1′-oxopropyl)propanoate and

ethyl
4,7-dihydro-1,3-dimethyl-4-(4′-fluorophenyl)-6-(1′-methylethyl)-1H-pyrazolo[3,4-b]pyridine-5-carboxylate.

A mixture of 197 g of product of Step 1, 497 g of product of Step 2 and 2.24 l of absolute ethanol is refluxed for 72 hours and allowed to cool, and the ethanol is evaporated at reduced pressure to obtain a yellow-orange oil. The obtained yellow-orange oil is divided into two approximately equal halves, and each half is dissolved in the minimum amount of 7:3 methyl t-butyl ether/ethyl acetate and chromatographed on 1 kg of 70-230 mesh ASTM silica gel. The fractions from both chromatographies containing at least substantially pure first product of this Step or a mixture of both products of this Step (as indicated by thin layer chromatography) are combined and evaporated at reduced pressure to obtain a mixture of both products of this Step, and the fractions from both chromatcgraphies containing at least substantially pure second product of this Step are combined and evaporated at reduced pressure to obtain the compound.

Step 4

Ethyl
4,7-dihydro-1,3-dimethyl-4-(4′-fluorophenyl)-6-(1′-methylethyl)-1H-pyrazolo[3,4-b]pyridine-5-carboxylate

88.4 ml of concentrated sulfuric acid is carefully added to a solution of 549.5 g of a mixture of both products of Step 3 in 3.5 l of absolute ethanol, and the reaction mixture is refluxed for 44 hours, allowed to cool to 20°-25°C and poured into 9 l of water. The mixture is carefully neutralized with sodium carbonate and extracted three times with 1.9 l portions of diethyl ether. The diethyl ether extracts are combined, dried over anhydrous magnesium sulfate, filtered and evaporated at reduced pressure to an oil. The obtained oil is dissolved in the minimum amount of 1:1 ethyl acetate/n-hexane and chromatographed on 2 kg of 70-230 mesh ASTM silica gel utilizing 1:1 ethyl acetate/n-hexane as the eluant. The fractions containing at least substantially pure product (as indicated by thin layer chromatography) are combined and evaporated at reduced pressure to obtain the crude product as an oil. Unreacted starting material may be isolated from other fractions and recycled.

Step 5 (Reaction BH)

Ethyl 1,3-dimethyl-4-(4′-flucrophenyl)-6-(1′-methylethyl)-1H-pyrazolo[3,4-b]pyridine-5-carboxylate

239 g of 2,3-dichloro-5,6-dicyanobenzoquinone is added over a period of 15-30 minutes to a solution of 63.5 g of second product of Step 3 and 308.5 g of crude product of Step 4 in 3 l of dioxane during which the internal temperature rises from 20°-25°C to 40°C, and the reaction mixture is stirred at 20°-25°C for 1.5-2 hours and poured into 8 l of saturated sodium bicarbonate solution. The basic mixture is stirred for 5 minutes at 20°-25°C and extracted once with 1.9 l of diethyl ether and twice with 1.4 l portions of diethyl ether. The diethyl ether extracts are combined, dried over anhydrous magnesium sulfate, filtered and evaporated at reduced pressure to obtain a dark red oil. The obtained dark red oil is chromatographed on 1.5 kg of 70-230 mesh ASTM silica gel utilizing methyl t-butyl ether as the eluant. The fractions containing a substantial amount of product (as indicated by thin layer chromatography) are combined and evaporated at reduced pressure to obtain a cherry red oil. The oil is dissolved in 800 ml of mixed hexanes, and the solution is maintained at 0°C for 16 hours and at -20°C for 30 minutes. The precipitate is collected by filtration, washed with 400 ml of cold (0°-5°C) n-hexane and dried to constant weight at 10-30 mm Hg and 20°-25°C to obtain the product (M.P. 68°-71°C).

Step 6 (Reaction AA)

1,3-Dimethyl-4-(4′-fluorophenyl)-6-(1′-methylethyl)-1H-pyrazolo[3,4-b]pyridine-5-methanol

1.8 l cf 1M. diisobutylaluminum hydride/n-hexane is added dropwise over a period of 1 hour to a solution of 90 g of product of Step 5 in 1.45 l of dry tetrahydrofuran stirred at 0°C while maintaining the temperature at -5° to 5°C, and the reaction mixture is allowed to warm to 20°-25°C, stirred at this temperature for 48 hours and cooled to 5°C, the reaction mixture being stirred under nitrogen throughout. 1.8 l of saturated ammonium chloride solution is added carefully with stirring at 5°C, 950 ml of diethyl ether is added, the mixture is filtered using some Celite$^R$ filter aid, the organic layer is separated, and the aqueous layer is extracted twice with 950 ml portions of diethyl ether. The diethyl ether extracts are combined with the previous organic layer, and the combined organic solution is dried over anhydrous magnesium sulfate, filtered and evaporated at reduced pressure to obtain a yellowish-white solid. The obtained solid is dissolved in 390 ml of hot methyl t-butyl ether, filtered through a cotton plug, cooled to 0°C and finally cooled to -20°C. The resulting solid is collected by filtration, washed with a little cold methyl t-butyl ether and vacuum dried at 50°C to obtain the product (M.P. 166°-168°C).

Step 7 (Reaction BA)

5-Chloromethyl-1,3-dimethyl-4-(4′-fluorophenyl)-6-(1′-methylethyl)-1H-pyrazolo[3,4-b]pyridine

A solution of 10.0 g of product of Step 6 and 12.0 g of thionyl chloride in 250 ml of methylene chloride is refluxed for 4 hours and cooled to 20°-25°C, and the excess thionyl chloride and methylene chloride are evaporated at reduced pressure. 100 ml of toluene is added to the residue, the toluene is evaporated at

reduced pressure, and these two steps are repeated twice to obtain the crude product as an orange-brown oil.

Step 8 (Reaction BC)

Dimethyl [[1,3-dimethyl-4-(4′-fluorophenyl)-6-(1′-methylethyl)-1H-pyrazolo[3,4-b]pyrid-5-yl]methyl]phosphonate

60 ml of trimethyl phosphite is carefully added to the crude product of Step 7 of this Example, stirred at 20°C, the reaction mixture is refluxed for 18 hours and cooled, and the excess trimethyl phosphite is evaporated at reduced pressure to obtain an orange oil which solidifies upon standing. The solid is triturated with mixed hexanes, collected by filtration and vacuum dried to obtain the product. An analytical sample is obtained by recrystallization from methyl t-butyl ether (M.P. 153°-154°C).

Step 9 (Reaction G)

1,1-Dimethylethyl (3R,5S)-(E)-7-[1′,3′-dimethyl-4′-(4″-fluorophenyl)-6′-(1″-methylethyl)-1H-pyrazolo[3,4-b]pyrid-5′-yl]-3,5-di-[(1′,1′-dimethylethyl)diphenylsilyloxy]hept-6-enoate

16 ml of 1.6M n-butyllithium/hexane is added rapidly dropwise to a solution of 10 g of product of Step 8 in 100 ml of dry tetrahydrofuran stirred at -78°C, the resulting orange solution is stirred at -78°C for 10 minutes, a solution of 18 g of 1,1-dimethylethyl (3R,5S)-3,5-di-[(1′,1′-dimethylethyl)diphenylsilyloxy]-6-oxohexanoate (product of Step 8′ hereunder) in 265 ml of dry tetrahydrofuran is added rapidly dropwise with stirring at -78°C, the resulting pale yellow solution is stirred at -78°C for 15 minutes, 20 ml of 1.5M lithium diisopropyl-amide bis(tetrahydrofuran) complex/cyclohexane is added via syringe with stirring at -78°C, and the reaction mixture is allowed to warm to 20°-25°C with stirring and is stirred at 20°-25°C for 30 minutes, the reaction mixture being stirred under argon throughout. The reaction mixture is poured into a mixture of 800 ml of water and 150 ml of saturated ammonium chloride solution, and the mixture is extracted three times with diethyl ether. The diethyl ether extracts are combined, dried over anhydrous magnesium sulfate, filtered and evaporated at reduced pressure to obtain an orange oil. The obtained oil is flash chromatographed on 230-400 mesh ASTM silica gel utilizing 4:1 mixed hexanes/methyl t-butyl ether as the eluant, and the fractions containing the product (as indicated by thin layer chromatography) are combined and evaporated at reduced pressure to obtain the product as a yellow-orange resin.

The compound used in Step 9 above, i.e. 1,1-dimethylethyl (3R,5S)-3,5-di-[(1′,1′-dimethylethyl)diphenylsilyloxy]-6-oxohexanoate, is obtained as follows:

Step 1′

Dimethyl (S)-hydroxybutanedioate

(a) 750 g of acetyl chloride is added over a period of 30 minutes to 13.5 l of methanol stirred at -5° to 0°C while maintaining an internal temperature of 0° to 2°C (the addition being very exothermic), the reaction mixture is stirred at 0°C for 1 hour, 1.76 kg of (S)-hydroxybutanedioic acid is added over a period of 30 minutes while maintaining an internal temperature of 0° to 2°C (the addition being slightly exothermic), and the reaction mixture is stirred at 0° to 2°C for 2 hours and at 20°-25°C for 16 hours, the reaction mixture being stirred under nitrogen throughout. 898 g of anhydrous sodium carbonate is added over a period of 15 minutes while maintaining an internal temperature of 20°-25°C to adjust the pH to 7-8 (the addition being slightly exothermic), and the reaction mixture is stirred at 20°-25°C for 30 minutes and filtered. The filter cake is washed twice with 500 ml portions of methanol, the filtrate and washings are combined, and the solution is evaporated at 25-30 mm Hg while maintaining an internal temperature of 35°-40°C to a volume of about 2.5 l and filtered. The filter cake is washed with 500 ml of methanol, the filtrate and washing are combined, and the solvent is distilled at 25-35 mm Hg and an internal temperature of 40°C to obtain the crude product.

(b) A solution of 300 g of crude product from Part (a) of this step in 300 ml of methylene chloride is added to a slurry of 300 g of 70-230 mesh ASTM silica gel in 1.4 l of methylene chloride, and the product is eluted with 7-8 l of methylene chloride. The methylene chloride is distilled at 30-40 mm Hg and an external temperature of 50°C to obtain the partially purified product which is vacuum distilled at 0.6 mm Hg and an internal temperature of 96°-98°C to obtain the product (B.P. 90°-92°C/1.5-2 mm Hg; 78°-80°C/0.6 mm Hg).

(c) The remainder of the crude product is similarly purified.

Step 2′

Methyl (S)-3,4-dihydroxybutanoate

7.5 g of sodium borohydride is added in one portion to a mixture of 523 ml of 10M boranedimethylsulfide complex and 1.8 l of dry tetrahydrofuran stirred at 20°-25°C, the reaction mixture is stirred at 20°-25°C for 30 minutes, a solution of 744 g of product of Step 1′ in 1.2 l of dry tetrahydrofuran is added over a period of 1 hour while maintaining with external cooling at 3°-5°C an internal temperature of 20°-25°C (the reaction is very exothermic, and hydrogen is evolved), and, after the exotherm stops, the reaction mixture is stirred at 20°-25°C for 1 hour and cooled to 20°C, the reaction mixture being stirred under nitrogen throughout. 1.8 l of

methanol is slowly added while maintaining an internal temperature of 20°-25°C (the first third of the addition is very exothermic and foaming occurs). The reaction mixture is stirred for 30 minutes while maintaining an internal temperature of 20°-25°C, the solvent is distilled at 20-30 mm Hg and an external temperature of 40°-45°C, 500 ml of toluene is added, the solvent is distilled at 20-30 mm Hg and an external temperature of 40°-45°C, an additional 500 ml of toluene is added, and the solvent is distilled at 20-30 mm Hg and an external temperature of 40°-45°C to obtain the product as a very thick stirrable oil. The entire reaction and the solvent distillations must be vented through bleach to trap any escaping dimethylsulfide.

Step 3′

Methyl (S)-3-hydroxy-4-(triphenylmethoxy)butanoate

(a) A solution of 1.82 kg of triphenylmethyl chloride in 2.5 l of methylene chloride is added over a period of 12 minutes to a mixture of 716 g of product of Step 2′, 880 g of triethylamine and 4.9 l of methylene chloride stirred at 15°C while maintaining an internal temperature of 15°-20°C (the addition being exothermic), and the reaction mixture is stirred at 23°C for 16 hours, stirred at an internal temperature of 38°-40°C for 15 minutes (during which gas evolves) and cooled to 25°C, the reaction mixture being maintained under nitrogen throughout. 7.4 l of water is added, the bottom organic layer is separated, the aqueous layer is extracted with 500 ml of methylene chloride, the methylene chloride extract is combined with the bottom organic layer, and the combined organic layers are washed successively with 7.4 l of water, 7.4 l of saturated sodium bicarbonate solution and 3.7 l of water and filtered to remove any insolubles. The solvent is distilled at 20-30 mm Hg and a maximum external temperature of 50°-60°C to obtain a thick dark stirrable oil. 2.5 l of methanol is added with stirring at 50°C, and the mixture is stirred at 50°C for 5 minutes, cooled to 20°C and filtered. The filter cake is washed with 1 l of methanol, the filtrate and washing are combined, and the solvent is distilled at 20-30 mm Hg and an external temperature of about 50°C to obtain the crude product as a thick stirrable brown oil.

(b) A 90 cm x 13 cm chromatographic column is charged with 7.2 l of 1:2 ethyl acetate/mixed hexanes, 450 g of sea sand is added, a slurry of 4 kg of 70-230 mesh ASTM silica gel in 12 l of 1:2 ethyl acetate/mixed hexanes is added while draining some of the solvent from the bottom of the column, sufficient solvent to lower the liquid level to the top of the silica gel is drained from the column, a solution of 1.0 kg of the crude product of Part (a) of this Step in 1.0 1 of methylene chloride is added, sufficient solvent to lower the liquid level to the top of the silica gel is drained from the column, the column is eluted with 18 l of 1:2 ethyl acetate/mixed hexanes to obtain six fractions, and the column is eluted with 12 l of 1:1 ethyl acetate/mixed hexanes to obtain eight fractions containing relatively pure product. These eight fractions are combined, the solvent is distilled at 20-30 mm Hg and 60°C to obtain a thick stirrable oil, 1 l of isopropanol is added, the solvent is distilled at 20-30 mm Hg and a maximum internal temperature of 60°C, an additional 1 l of isopropanol is added, the solvent is distilled at 20-30 mm Hg and a maximum internal temperature of 60°C, and the residue is added to 2 l of isopropanol. The resulting solution is cooled to 0° to 5°C and maintained at this temperature for 3-4 hours. The solid is collected by filtration, washed twice with 250 ml-portions of cold (0°-5°C) isopropanol and vacuum dried at 50°-60°C to constant weight to obtain the product [M.P. 80°-82°C; $[\alpha]^{25}_{589}$ = -5.52° (c = 1.5580, $CH_2Cl_2$)].

All chromatography fractions and mother liquors containing impure product may be combined and rechromatographed, etc. to obtain additional product.

(c) The balance of the crude product is similarly purified.

Step 4′

1,1-Dimethylethyl (S)-5-hydroxy-3-oxo-6-(triphenylmethoxy)hexanoate

1.98 l of 1.6M n-butyllithium/hexane is added over a 30 minute period to a mixture of 321 g of diisopropylamine and 1.5 l of dry tetrahydrofuran stirred at -50° to -40°C, during which the internal temperature rises to -5° to 0°C, the pale yellow solution is stirred at -5° to 0°C for 30 minutes and cooled to -65°C, 367 g of t-butyl acetate is added over a period of 40 minutes while maintaining an internal temperature of -63° to -60°C (the addition being exothermic), the reaction mixture is stirred at -62° to -60°C for 40 minutes, a solution of 300 g of product of Step 3′ in 1.35 l of dry tetrahydrofuran is added over a period of 25 minutes while maintaining an internal temperature of -62° to -58°C (the addition being exothermic), and the reaction mixture is stirred at -62° to -58°C for 1 hour, warmed to -5° to 0°C over a period of 45 minutes and stirred at -5° to 0°C for 30 minutes, the reaction mixture being stirred under nitrogen throughout. 1.8 l of saturated ammonium chloride solution is added over a period of 3 minutes while maintaining an internal temperature of below 15°C (the addition being exothermic), the mixture is stirred at 10°-15°C for 10 minutes, 2.1 l of toluene is added, and the top organic layer is separated, washed three times with 1.5 l portions of water and, if any insolubles are present, filtered. The solvent is distilled at 20-30 mm Hg and an internal temperature of 50°-60°C to obtain the crude product as a very thick yellow oil that is stirrable at 50°C.

Step 5′

1,1-Dimethylethyl (3R,5S)-3,5-dihydroxy-6-(triphenylmethoxy)hexanoate

A mixture of 715 ml of 1M triethylborane/tetrahydrofuran, 4.76 l of dry tetrahydrofuran and 1.44 l of methanol is stirred at 18°-22°C for 45 minutes and cooled to -75°C, a solution of 254.4 g of crude product of Step 4′ in

960 ml of dry tetrahydrofuran is added over a period of 15 minutes while maintaining an internal temperature of -75° to -72°C, the reaction mixture is stirred at -76° to -75°C for 1 hour, 24.96 g of sodium borohydride is added in portions over a period of 15 minutes while maintaining an internal temperature of -76° to -72°C, and the reaction mixture is stirred at -77° to -76°C for 4.5 hours, the reaction mixture being stirred under nitrogen throughout. 756 ml of saturated ammonium chloride solution is slowly added over a period of 25 minutes while maintaining an internal temperature of -76° to -67°C (the addition is exothermic, and some foaming occurs), the mixture is stirred at -68° to -67°C for 30 minutes and warmed to 10°-15°C over a period of 30 minutes, 360 ml of water is added, the mixture is extracted with 3.6 l of ethyl acetate, the top organic layer is separated and washed successively with 750 ml of a 1:1 mixture of water and saturated sodium chloride solution and 750 ml of saturated sodium chloride solution, and the solvent is distilled at 20-30 mm Hg and a maximum external temperature of 50°C. 180 ml of ethyl acetate is added, the solvent is distilled at 20-30 mm Hg and a maximum external temperature of 50°C, and these two steps are repeated once. 2.91 l of ethyl acetate is added to the obtained thick oil (the cyclic boronate), 290 ml of 30% hydrogen peroxide solution is slowly added over a period of 20-30 minutes while maintaining an internal temperature of 20°-25°C (the addition initially being exothermic), and the reaction mixture is stirred at 20°-25°C for 2.5 hours and added to 360 ml of water. The top organic layer is separated, washed three times (for 10-15 minutes each time) with 480 ml portions of cold (0°-5°C) 10% sodium sulfite solution (until the organic layer is free of peroxide) while maintaining an internal temperature of 25°C and washed successively with 480 ml of saturated sodium bicarbonate solution, 480 ml of water (any emulsion that forms will separate within about an hour) and 480 ml of saturated sodium chloride solution. The top organic layer is separated, and the solvent is distilled at 20-30 mm Hg and a maximum external temperature of 50°-60°C. 326 ml of methanol is added, the solvent is distilled at 20-25 mm Hg and an internal temperature of 50°-60°C, and these two steps are repeated four more times. 100 ml of toluene is added, the solvent is distilled at 20-30 mm Hg and an internal temperature of 60°C, and these two steps are repeated three more times. The obtained oil is heated for an additional 3-4 hours at 20-30 mm Hg and 50°-60°C to obtain the product (the ratio of the 3R,5S isomer to the 3S,5S isomer is about 69:1 although in other batches it was as low as about 23:1.)

Step 6′

1,1-Dimethylethyl (3R,5S)-3,5-di-[(1′,1′-dimethylethyl)diphenylsilyloxy]-6-(triphenylmethoxy)hexanoate

132.5 g of imidazole is added to a mixture of 267 g of product of Step 5′ and 900 ml of N,N-dimethylformamide while maintaining an internal temperature of 23°-25°C, the mixture is heated to 70°-72°C, 235.8 g of t-butyldiphenylchlorosilane is added over a period of 2 minutes, and the reaction mixture is stirred at 70°-72°C for 18 hours and cooled to 10°C, the reaction mixture being stirred under nitrogen throughout. The reaction mixture is added to a mixture of 2.5 l of hexane and 1.1 l of water the mixture is stirred for 5 minutes, the organic top layer is separated and washed twice with 1.1 l portions of water, and the solvent is distilled at 20-30 mm Hg and an external temperature of 40°-60°C to obtain the product.

Step 7′

1,1-Dimethylethyl (3R,5S)-3,5-di-[(1′,1′-dimethylethyl)diphenylsilyloxy]-6-hydroxyhexanoate

(a) A cold solution prepared from 960 g of water, 600 g of ice and 740 g of trifluoroacetic acid is added over a period of 2 minutes to a solution of 432 g of product of Step 6′ in 3.4 l of methylene chloride while maintaining a maximum internal temperature of 25°C, and the reaction mixture is stirred at 22°-26°C for 2.5 hours, the reaction mixture being stirred under nitrogen throughout. The organic bottom layer is separated and washed successively with 1.1 l of saturated sodium bicarbonate solution and 1.0 l of water, and the solvent is distilled at 20-30 mm Hg and a maximum external temperature of 60°C. 100 ml of mixed hexanes is added to the residue, the solvent is distilled at 20-30 mm Hg and a maximum internal temperature of 60°C, and these two steps are repeated once, the distillation being continued until no more solvent distills off. 1.1 l of mixed hexanes is added to the obtained thick semi-solid, the mixture is heated at 40°-45°C for 10-15 minutes, cooled to 0°-5°C and maintained at 0°-5°C for 10 minutes, and the solid is collected by filtration and washed twice with 100 ml portions of cold (10°C) mixed hexanes. The filtrate and hexane washings are combined, and the solvent is distilled at 20-30 mm Hg and an external temperature of 40°-60°C to obtain the crude product as an oil.

(b) The crude product of Part (a) is purified by HPLC using a Kiloprep 250 Millipore Process HPLC system with a reverse phase Kiloprep 250 $C_{18}$ cartridge (80 microns) and a 200 nm detector. The column is flashed with methylene chloride until it is decontaminated (as indicated by the UV detector) and flushed with methanol for about 10 minutes and then with 9:1 methanol/water for about 6 minutes at a flow rate of 0.5 l/min. A solution of the crude product of Part (a) of this Step in a mixture of 393 ml of methanol and 22 ml of water is placed on the column, and the column is eluted with 9:1 methanol/water for about 22 minutes, 11 minutes and 3 minutes to obtain fractions 1-3 and with methanol for about 4 minutes and 7 minutes to obtain fractions 4 and 5, the flow rate being 0.5 l/min. Fractions 4 and 5 are combined, the solvent is distilled at 20-40 mm Hg and a maximum internal temperature of 40°C, 250 ml of methylene chloride is added to the residue, the mixture is stirred at 30°C for 5 minutes, the organic bottom layer is separated, and the solvent is distilled at 20-30 mm Hg and a maximum internal temperature of 40°-45°C. 100 ml of n-heptane is added, the solvent is distilled at 20-30 mm Hg and 40°-45°C, and these two steps are repeated once. 460 ml of n-heptane is added to the residue, and the

mixture is heated to 80°-90°C to dissolve the oil, allowed to cool to 20°-25°C with stirring until solid forms and maintained at 10°C for 2-3 hours. The resulting white solid is collected by filtration, washed twice with 50 ml portions of cold (10°C) n-heptane and vacuum dried to constant weight at 50°C for 15 hours to obtain the product as a white solid [M.P. 79°-80°C; $[\alpha]^{20}_{589} = +8.35°$ (c = 5.0, $CH_2Cl_2$)] (the ratio of the 3R,5S isomer to the 3S,5S isomer is about 76:1.).

Step 8′

1,1-Dimethylethyl (3R,5S)-3,5-di-[(1′,1′-dimethylethyl)diphenylsilyloxy]-6-oxohexanoate

150 g of pyridinium chlorochromate is added over a period of 3 minutes to a mixture of 200 g of product of Step 7′, 2 l of methylene chloride and 400 g of 4Å powdered molecular sieves stirred at 20°C while maintaining an internal temperature of 20°-22°C (the addition being slightly exothermic), and the reaction mixture is vigorously stirred for 1 hour at 22°-25°C, the reaction mixture being stirred under nitrogen throughout. 3.32 l of n-heptane is added, and the mixture is stirred at 23°C for 5 minutes and filtered through 250 g of Celite[R] filter aid pre-wet with n-heptane. The filter cake is washed twice with 200 ml portions of n-heptane, the filtrate and the n-heptane washings are combined, and the solvent is distilled at 20-30 mm Hg and a maximum external temperature of 45°-50°C. 100 ml of n-heptane is added, the solvent is distilled at 20-30 mm Hg and a maximum external temperature of 45°-50°C, and this is repeated once. The resulting oil is dissolved in 400 ml of 1:12 ethyl acetate/mixed hexanes and chromatographed on 420 g of 70-230 mesh ASTM silica gel topped with about 2.5 cm of sea sand utilizing a column having a 7 cm inside diameter and 4 l of 1:12 ethyl acetate/mixed hexanes as the eluant. The solvent is distilled at 20-30 mm Hg and an external temperature of 50°-55°C. 100 ml of n-heptane is added, the solvent is distilled at 20-30 mm Hg and an external temperature of 50°-55°C, this is repeated once, and the residue is maintained at this pressure and temperature for 2 hours. 335 ml of mixed hexanes is added at 40°-50°C to the obtained thick oil, and the mixture is cooled to -35° to -30°C, seeded and maintained at -35° to -30°C for 1 hour. The resulting white solid is collected by filtration, washed twice with 50 ml portions of cold (-25° to -20°C) mixed hexanes and dried to constant weight at 20-30 mm Hg and 45°-50°C for 24 hours to obtain the product [M.P. 81°-82°C.; $[\alpha]^{25}_{589} = +5.21°$ (c = 5.0, $CH_2Cl_2$)] (the ratio of the 3R,5S isomer to the 3S,5S isomer is about 99:1.).

## Example 2

**Sodium (3R,5S)-(E)-3,5-dihydroxy-7-[1′,3-dimethyl-4′-(4″-fluorophenyl)-6′-(1″-methylethyl)-1H-pyrazolo[3,4-b]pyrid-5′-yl]hept-6-enoate**

[hydrolysis; process variant b); reaction K)

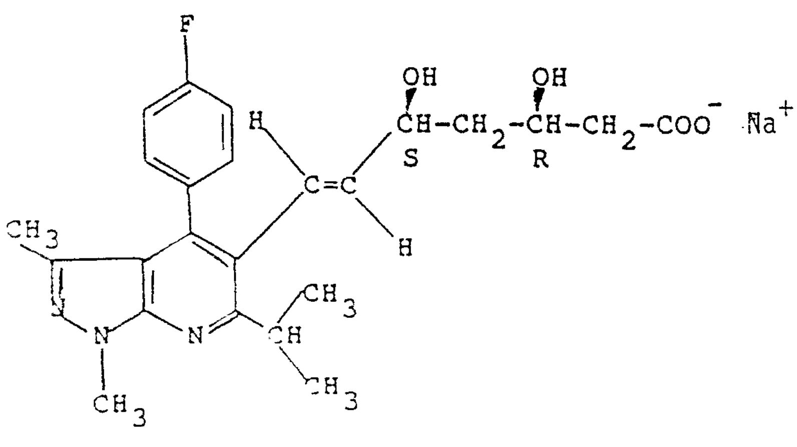

A mixture of 8.3 g of title product of Example 1, 16.5 ml of 1N sodium hydroxide solution and 150 ml of ethanol is stirred at 20°-25°C under nitrogen for 3.5 hours, the solvent is evaporated at reduced pressure to obtain a brown foamy resin, about 75 ml of diethyl ether is added, the inside wall of the reaction flask is rinsed with about 15 ml of chloroform so as to triturate the foamy resin, and the resulting yellow light brown powder is collected by filtration and vacuum dried for 24 hours to obtain the product [M.P. 199°-203°C (dec.)].
N.M.R. ($CD_3OD$):

1.16-1.40 (m, 1H), 1.3-1.34 (d, 6H), 1.42-1.61 (m, 1H), 1.90 (s, 3H), 2.10-2.32 (m, 2H), 3.48-3.53 (m, 1H), 3.63-3.78 (m, 1H), 4.02 (s, 3H), 4.19-4.29 (m, 1H), 5.33-5.44 (dd, 1H), 6.47- 6.56 (d, 1H), 7.11-7.38 (m, 4H)

## Example 3

**Ethyl (±)-erythro-(E)-3,5-dihydroxy-7-[1′,6′-dimethyl-4′-(4″-fluorophenyl)-3′-phenyl-1H-pyrazolo[3,4-b]pyrid-5′-yl]hept-6-enoate**

[reduction; process variant c); reaction B]

(a) 1.7 ml of 1.0M triethylborane/tetra-hydrofuran is added rapidly dropwise to a solution of 0.7 g of ethyl (±)-(E)-7-[1′,6′-dimethyl-4′-(4″-fluorophenyl)-3′-phenyl-1H-pyrazolo[3,4-b]pyrid-5′-yl]-5-hydroxy-3-oxohept-6-enoate (product of Step 10 hereunder) in 5 ml of 4:1 tetrahydrofuran/methanol stirred at 20°-25°C, the reaction mixture is stirred at 20°-25°C for 1 hour and cooled to -70°C, 0.06 g of sodium borohydride is added in one portion, and the reaction mixture is stirred at -70°C for 3 hours, the reaction mixture being stirred under nitrogen throughout. The reaction mixture is quenched with 4 ml of saturated ammonium chloride solution and allowed to warm to 20°-25°C, sufficient water to dissolve all solids is added, and the mixture is extracted twice with diethyl ether. The diethyl ether extracts are combined, dried over anhydrous magnesium sulfate, filtered and evaporated at reduced pressure to obtain the cyclic boron ester.

(b) A solution of the product of Part (a) of this step in 5 ml of methanol is stirred at 20°-25°C for 16 hours and evaporated at reduced pressure, and the residue is dissolved in diethyl ether. The diethyl ether solution is washed twice with saturated sodium chloride solution, dried over anhydrous magnesium sulfate, filtered and evaporated at reduced pressure, and the residual oil is chromatographed on 230-400 mesh ASTM silica gel utilizing 3:2:1 ethyl acetate/ methyl t-butyl ether/mixed hexanes and then ethyl acetate as the eluent. The fractions containing the product (as indicated by thin layer chromatography) are combined and evaporated at reduced pressure to obtain the product:

N.M.R. ($CDCl_3$):

1.26-1.32 (t, 3H), 2.39-2.47 (m, 2H), 2.72 (s, 3H), 4.20 (s, 3H), 4.2S-4.43 (m, 1H), 5.27-5.38 (dd, 1H), 6.46-6.54 (d, 1H), 6.68-6.83 (t, 2H), 6.86-7.20 (m, 7H)

The product is at least 95% pure erythro racemate; any threo racemate present therein may be separated therefrom by conventional means. The erythro racemate may be resolved into two optically pure enantiomers, the 3R,5S and 3S,5R enantiomers, of which the former is preferred. Any threo racemate may be resolved to obtain the 3R,5R and 3S,5S enantiomers. The use of a non-stereoselective reduction would afford a mixture of all four stereoisomers wherein the ratio of the erythro stereoisomers to the threo stereoisomers ranges from 3:2 to 2:3.

The starting material is obtained as follows:

### Step 1

5-Amino-1-methyl-3-phenylpyrazole

A mixture of 11.0 g of benzoylacetonitrile, 3.5 g of methylhydrazine and 25 ml of isopropanol is refluxed under nitrogen for 24 hours, allowed to cool to 20°-25°C and evaporated at reduced pressure. The residual oil is triturated with toluene, and the obtained solid is collected by filtration and vacuum dried to obtain the product as a yellow powder (M.P. 124°-128°C).

### Step 2

Methyl (Z)-2-[(4′-fluorophenyl)methylene]-3-oxobutanoate

A mixture of 100.0 g of 4-fluorobenzaldehyde, 93.6 g of methyl acetoacetate, 7 ml of glacial acetic acid, 7.0 g of piperidine and 900 ml of toluene is refluxed for 16 hours to remove the water that forms, allowed to cool to 20°-25°C, washed successively four times with 125 ml portions of 5% sodium hydroxide solution, four times with 125 ml portions of 10% sodium bisulfite solution and four times with 250 ml portions of water, dried over anhydrous magnesium sulfate, filtered and evaporated at reduced pressure. The residue is chromatographed on 230-400 mesh ASTM silica gel utilizing 1:1 ethyl acetate/mixed hexanes as the eluant. The fractions containing the product (as indicated by thin layer chromatography) are combined, evaporated at reduced pressure and vacuum distilled at 0.18 mm Hg to obtain the crude product as a dense yellow oil (B.P. 114°-115°C/0.18 mm Hg) (the obtained crude product contains two components according to thin layer chromatography, the second probably being the corresponding (E) compound).

### Step 3

Methyl 4,7-dihydro-1,6-dimethyl-4-(4′-fluorophenyl)-3-phenyl-1H-pyrazolo[3,4-b]pyridine-5-carboxylate

A mixture of 5.3 g of product of Step 1, 6.2 g of product of Step 2 and 25 ml of HPLC grade methanol is refluxed under nitrogen for 16 hours and stirred at 20°-25°C for 64 hours. The solidified mixture is dissolved in 20 ml of HPLC grade methanol and refluxed under nitrogen for 72 hours, allowed to cool to 20°-25°C and evaporated at reduced pressure. The residue is triturated with diethyl ether, and the obtained solid is collected by filtration and vacuum dried to obtain the product. The filtrate is evaporated at reduced pressure, and the residue is flash chromatographed on 230-400 mesh ASTM silica gel utilizing methylene chloride to elute two contaminants and ethyl acetate to elute the product. The fractions containing the product (as indicated by thin layer chromatography) are combined and evaporated at reduced pressure, and the residue is triturated with diethyl ether to obtain additional product (M.P. 181°-183°C).

Step 4 (Reaction BH)

Methyl 1,6-dimethyl-4-(4′-fluorophenyl)-3-phenyl-1H-pyrazolo[3,4-b]pyridine-5-carboxylate

A mixture of 6.6 g of product of Step 3, 4.0 g of 2,3-dichloro-5,6-dicyano-1,4-benzoquinone and 20 ml of dry tetrahydrofuran is stirred at 20°-25°C for 1 hour, an additional 0.4 g of 2,3-dichloro-5,6-dicyano-1,4-benzoquinone is added, and the reaction mixture is stirred at 20°-25°C for 1 hour, the reaction mixture being stirred under nitrogen throughout. The solvent is evaporated at reduced pressure, and the residue is flash chromatographed on 230-400 mesh ASTM silica gel utilizing 1:1 methyl t-butyl ether/mixed hexanes as the eluant. The fractions containing the pure product (as determined by thin layer chromatography) are combined and evaporated at reduced pressure, and the residue is triturated with mixed hexanes. The obtained solid is collected by filtration and vacuum dried to obtain the product (M.P. 142°-144°C).

Step 5 (Reaction AA)

1,6-Dimethyl-4-(4′-fluorophenyl)-3-phenyl-1H-pyrazolo-[3,4-b]pyridine-5-methanol

48.0 ml of 1M diisobutylaluminum hydride is added rapidly dropwise to a solution of 4.5 g of product of Step 4 in 35 ml of dry tetrahydrofuran stirred at 0°C, and the reaction mixture is allowed to warm to 20°-25°C, stirred at 20°-25°C for 4 hours and cooled to 0°C, the reaction mixture being stirred under nitrogen throughout. The reaction mixture is quenched with saturated ammonium chloride solution, acidified to about pH 6 with 2N hydrochloric acid and extracted three times with methyl t-butyl ether. The methyl t-butyl ether extracts are combined, dried over anhydrous magnesium sulfate, filtered and evaporated at reduced pressure. The residue is triturated with mixed hexanes to obtain the yellow crystalline crude product. The aqueous layer from the methyl t-butyl ether extractions is extracted with methylene chloride, the methylene chloride extract is evaporated at reduced pressure, and the residue is triturated with mixed hexanes to obtain additional product as white crystals (M.P. 231°-236°C).

Step 6 (Reaction AB)

1,6-Dimethyl-4-(4′-fluorophenyl)-3-phenyl-1H-pyrazolo[3,4-b]pyridine-5-carboxaldehyde

A mixture of 2.4 g of product of Step 5, 10.0 g of activated manganese dioxide and 25 ml of toluene is refluxed for 16 hours, allowed to cool to 20°-25°C and filtered through Celite[R] filter aid. The Celite[R] is rinsed with diethyl ether, the diethyl ether rinse is combined with the toluene filtrate, the solvent is evaporated at reduced pressure, the residue is triturated with mixed hexanes, and the solid is collected by filtration and vacuum dried to obtain the product.

Step 7 (Reaction AF)

Ethyl (E)-3-[1′,6′-dimethyl-4′-(4″-fluorophenyl)-3′-phenyl-1H-pyrazolo[3,4-b]pyrid-5′-yl]propenoate

0.3 g of 60% sodium hydride/mineral oil is washed with mixed hexanes and suspended in 3 ml of dry tetrahydrofuran, the suspension is cooled to 0°C, a solution of 1.5 ml of triethylphosphonoacetate in 5 ml of dry tetrahydrofuran is added rapidly dropwise, the mixture is stirred at 0°C for 1 hour, a solution of 1.7 g of product of Step 6 in 17 ml of dry tetrahydrofuran is added rapidly dropwise, and the reaction mixture is allowed to warm to 20°-25°C and stirred at this temperature for 16 hours, the reaction mixture being stirred under nitrogen throughout. The reaction mixture is poured into water, and the mixture is extracted three times with diethyl ether. The diethyl ether extracts are combined, dried over anhydrous magnesium sulfate, filtered and evaporated at reduced pressure. The residue is triturated with cold mixed hexanes, and the resulting solid is collected by filtration and vacuum dried to obtain the crude product.

Step 8 (Reaction AG)

(E)-3-[1′,6′-Dimethyl-4′-(4″-fluorophenyl)-3′-phenyl-1H-pyrazolo[3,4-b]pyrid-5′-yl]prop-2-en-1-ol

14.4 ml of 1M diisobutylaluminum hydride/tetrahydrofuran is added rapidly dropwise to a solution of 1.5 g of product of Step 7 in 10 ml of dry tetrahydrofuran stirred at 0°-5°C, and the reaction mixture is allowed to warm to 20°-25°C and stirred at 20°-25°C for 16 hours, the reaction mixture being stirred under nitrogen

throughout. The reaction mixture is quenched with saturated ammonium chloride solution, acidified to about pH 6 with 2N hydrochloric acid and extracted three times with diethyl ether. The diethyl ether extracts are combined, dried over anhydrous magnesium sulfate, filtered and evaporated at reduced pressure to obtain the crude product as a white powder.

Step 9 (Reaction AH)

(E)-3-[1′,6′-Dimethyl-4′-(4″-fluorophenyl)-3′-phenyl-1H-pyrazolo[3,4-b]pyrid-5′-yl]prop-2-en-1-al

A mixture of 1.2 g of product of Step 8, 4.8 g of activated manganese dioxide and 40 ml of toluene is refluxed for 16 hours and, while hot, filtered through Celite[R] filter aid. The Celite[R] is washed with 1:1 diethyl ether/methylene chloride, and the washing is combined with the previous filtrate. The combined organic solution is evaporated at reduced pressure, and the residue is triturated with cold mixed hexanes. The resulting solid is collected by filtration and vacuum dried to obtain the crude product. Additional crude product may be obtained from the mother liquor.

Step 10 (Reaction A)

Ethyl
(±)-(E)-7-[1′,6′-dimethyl-4′-(4″-fluorophenyl)-3′-phenyl-1H-pyrazolo[3,4-b]pyrid-5′-yl]-5-hydroxy-3-oxohept-6-enoate

(a) 0.6 g of sodium hydride/mineral oil is rinsed with hexane and suspended in 10 ml of dry tetrahydrofuran, a solution of 1.9 ml of ethyl acetoacetate in 5 ml of dry tetrahydrofuran is added dropwise with stirring at 0° C, the reaction mixture is stirred at 0° C for 20 minutes, 9.6 ml of 1.55M n-butyllithium/hexane is added dropwise with stirring at 0° C, and the reaction mixture is stirred at 0° C for 20 minutes and cooled to -20° C, the reaction mixture being stirred under nitrogen throughout.

(b) about 60% of the dianion solution of Part (a) of this step is added via syringe to a solution of 1.1 g of crude product of Step 9 in 15 ml of dry tetrahydrofuran stirred at -20° C, and the reaction mixture is stirred at -20° C for 20 minutes, the reaction mixture being stirred under nitrogen throughout. The reaction mixture is poured into a mixture of 50 ml of saturated ammonium chloride solution and 50 ml of water, and the mixture is extracted three times with diethyl ether. The diethyl ether extracts are combined, dried over anhydrous magnesium sulfate, filtered and evaporated at reduced pressure. The residue is flash chromatographed on 230-400 mesh ASTM silica gel utilizing 1:1 methyl t-butyl ether/mixed hexanes as the eluant, and the fractions containing the product (as indicated by thin layer chromatography) are combined and evaporated at reduced pressure to obtain the product as a yellow solid.

## Example 4

**Sodium**
**(±)-erythro-(E)-3,5-dihydroxy-7-[1′,6′-dimethyl-4′-(4″-fluorophenyl)-3′-phenyl-1H-pyrazolo[3,4-b]pyrid-5′-yl]hept-6-enoate**

[hydrolysis; process variant b); reaction K]

The product [M.P. 210°-225° C (dec.)] is obtained in a manner analogous to Example 2, starting from the title product of Example 3; N.M.R. ($CD_3OD$):
1.25-1.41 (m, 1H), 1.49-1.66 (m, 1H), 2.12-2.33 (m, 2H), 2.74 (s, 3H), 3.71-3.88 (m, 1H), 4.17 (s, 3H), 5.34-5.48 (dd, 1H), 6.42-6.52 (d, 1H), 6.72-7.23 (m, 9H)

The product is at least 95% pure erythro racemate; any threo racemate present therein may be separated therefrom by conventional means. The erythro racemate may be resolved into two optically pure enantiomers, the 3R,5S and 3S,5R enantiomers, of which the former is preferred.
Any threo racemate may be resolved to obtain the 3R,5R and 3S,5S enantiomers. The use of a starting material synthesized by using a non-stereoselective reduction in Example 3 would afford a mixture of all four stereoisomers wherein the ratio of the erythro stereoisomers to the threo stereoisomers ranges from 3:2 to 2:3.

TABLE I

**Examples 5 to 9**

The following additional compounds of Groups IAa and ICa may be synthesized by the processes set forth abov

| | $R_1$ | $R_2$ | $R_3$ | $R_4$ | X | $R_{17}$ | $R_{18}$ | Isomers | M.P. |
|---|---|---|---|---|---|---|---|---|---|
| Ex. 5 | $CH_3$ | 4-fluorophenyl | $CH_3$ | $CH_3$ | (E)-CH=CH- | H | $C_2H_5$ | E | Solid foam |
| Ex. 6 | $CH_3$ | 4-fluorophenyl | $CH_3$ | $CH_3$ | (E)-CH=CH- | H | $Na^+$ | E | Solid foam |
| Ex. 7 | i-$C_3H_7$ | 4-fluorophenyl | $CH_3$ | $CH_3$ | (E)-CH=CH- | H | $C_2H_5$ | E | Oil |
| Ex. 8 | i-$C_3H_7$ | 4-fluorophenyl | $CH_3$ | $CH_3$ | (E)-CH=CH- | H | $Na^+$ | E | Solid foam |
| Ex. 9 | i-$C_3H_7$ | cyclohexyl | $CH_3$ | $CH_3$ | (E)-CH=CH- | H | $C_2H_5$ | E | Oil |

E = erythro racemate (≧95% pure; balance, if any, threo racemate and/or impurities)

TABLE II

**Example 10**

The following compound of Group IAb may be synthesized by the processes set forth above:

| | $R_1$ | $R_2$ | $R_3$ | $R_4$ | X | $R_{17}$ | Isomers | M.P. |
|---|---|---|---|---|---|---|---|---|
| **Ex. 10** | **$CH_3$** | **4-fluorophenyl** | **$CH_3$** | **$CH_3$** | **(E)-CH=CH-** | **H** | **trans** | **Solid foam** |

trans = trans lactone racemate (≧95% pure; balance, if any, cis lactone racemate and/or impurities)

N.M.R. Data

Ex. 5 ($CDCl_3$):
1.26-1.34 (t, 3H), 1.93 (s, 3H), 2.41-2.46 (m, 2H), 2.68 (s, 3H), 4.07 (s, 3H), 4.13-4.23 (q, 2H), 4.28-4.41 (m, 1H), 5.30-5.40 (dd, 1H), 6.36-6.46 (d, 1H), 7.04-7.27 (m, 4H)
Ex. 6 ($CD_3OD$):
1.28-1.37 (m, 1H), 1.46-1.64 (m, 1H), 1.88 (s, 3H), 2.11-2.32 (m, 2H), 2.69 (s, 3H), 4.01 (s, 3H), 4.15-4.30 (m, 1H), 5.37-5.48 (dd, 1H), 6.36-6.47 (d, 1H), 7.12-7.37 (m, 4H)
Ex. 7 ($CDCl_3$):
1.92 (s, 3H), 2.38-2.46 (m, 2H), 3.32-3.49 (m, 1H), 4.06 (s, 3H), 4.13-4.25 (q, 2H), 4.23-4.41 (m, 1H), 5.22-5.33 (dd, 1H), 6.46-6.53 (d, 1H), 7.00-7.30 (m, 4H)
Ex. 8 ($CDCl_3$ + $CD_3OD$):
1.29-1.33 (d, 6H), 1.91 (s, 3H), 2.12-2.37 (m, 2H), 3.37-3.54 (m, 1H), 3.81-3.96 (m, 1H), 4.04 (s, 3H), 5.26-5.37 (dd, 1H), 6.42-6.51 (d, 1H), 7.04-7.28 (m, 4H)
Ex. 9 ($CDCl_3$):
1.15-1.4 (m, 9H), 1.58-2.10 (m, 10H), 2.73 (s, 3H), 3.22-3.45 (m, 1H), 4.01 (s, 3H), 4.10-4.27 (q, 2H), 4.32-4.47 (m, 1H), 5.64-5.78 (m, 1H), 6.80-6.89 (d, 1H)
Ex. 10 ($CDCl_3$):
1.93 (s, 3H), 2.50-2.78 (m, 2H), 2.68 (s, 3H), 4.07 (s, 3H), 4.18-4.29 (m, 1H), 5.04-5.17 (m, 1H), 5.32-5.43 (dd, 1H), 6.46-6.54 (d, 1H), 7.06-7.28 (m, 4H)

Any threo racemate present in Examples 5-9 may be isolated therefrom and resolved to obtain the 3R,5R and 3S,5S enantiomers, and each erythro racemate may be resolved to obtain the 3R,5S and 3S,5R enantiomers, of which in each case the former is preferred.

Any cis racemate present in Example 10 may be isolated therefrom and resolved to obtain the 4R,6R and 4S,6S enantiomers, and the trans racemate may be resolved to obtain the 4R,6S and 4S,6R enantiomers, the former being preferred in each case.

Each of the compounds of the Examples wherein Z is a group of Formula a wherein $R_{18}$ is a cation may be converted into the corresponding compounds wherein $R_{18}$ is hydrogen or a different cation M, particularly the latter, especially M′, by the processes set forth in Reaction Scheme III.

Each of examples 1-10 (including each of the possible optical isomers of each example) may be administered to inhibit cholesterol biosynthesis and thereby lower the blood cholesterol level for, for example, the treatment of atherosclerosis and hyperlipoproteinemia. The dosages are those set forth supra.

Throughout the specification, the term "reduced pressure" denotes aspirator pressure. Where no solvent is specified in connection with a solution, the solvent is water, and all solvent mixtures are by volume. When a reaction is carried out under nitrogen or argon, dry nitrogen or argon, as the case may be, is used to maintain anhydrous conditions (except where the reaction medium contains water).

All nuclear magnetic resonance spectra were taken at ambient temperature on a 200 MHz. spectrometer. All chemical shifts are given in p.p.m. (δ) relative to tetramethylsilane, and where a single δ value is given for anything other than a sharp singlet, it is its center point. In the N.M.R. data:
d = double
dd = doublet of a doublet
m = multiplet
q = quartet
s = singlet
t = triplet

**Claims**

1. A compound of Formula I

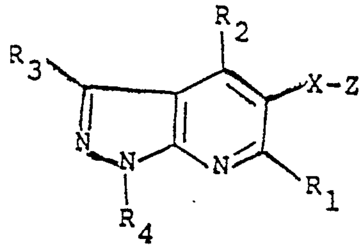

(I),

wherein $R_1$ is $C_{1-6}$alkyl not containing an asymmetric carbon atom, $C_{5-7}$cycloalkyl, ($C_{5-7}$cycloalkyl)methyl, phenyl-$(CH_2)_m$-, pyridyl-2, pyridyl-3, pyridyl-4, thienyl-2, thienyl-3 or Ring A

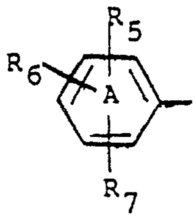
,

$R_2$ is $C_{1-6}$alkyl not containing an asymmetric carbon atom, $C_{5-7}$cycloalkyl, ($C_{5-7}$cycloalkyl)methyl, phenyl-$(CH_2)_m$-, pyridyl-2, pyridyl-3, pyridyl-4, thienyl-2, thienyl-3 or Ring B

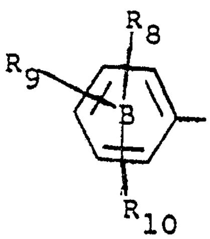
,

with the proviso that not more than one of $R_1$ and $R_2$ is a member of the group consisting of pyridyl-2, pyridyl-3, pyridyl-4, thienyl-2, thienyl-3, Ring A and Ring B,
$R_3$ is $C_{1-6}$alkyl not containing an asymmetric carbon atom, $C_{5-7}$cycloalkyl or Ring C

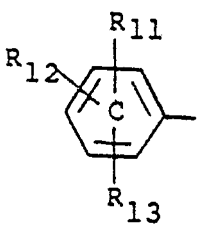
,

$R_4$ is $C_{1-6}$alkyl not containing an asymmetric carbon atom, $C_{5-7}$cycloalkyl or Ring D

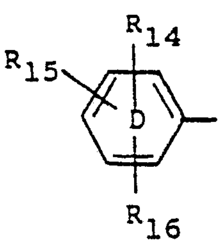
,

X is $-(CH_2)_n-$, $-CH=CH-$, $-CH_2-CH=CH-$ or $-CH=CH-CH_2-$,
wherein n is 1, 2 or 3, and

Z is 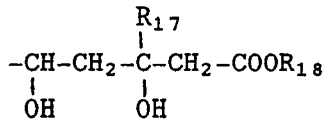 (a)

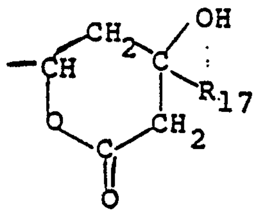 (b) or

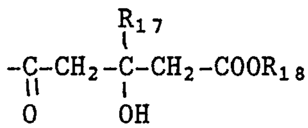 (c),

wherein $R_{17}$ is hydrogen or $C_{1-3}$alkyl, and $R_{18}$ is hydrogen, $R_{19}$ or M,
wherein $R_{19}$ is an ester group, and M is a cation,
with the proviso that Z may be a group of Formula c only when
(i) X is -CH=CH- or -$CH_2$-CH=CH- and/or
(ii) $R_{17}$ is $C_{1-3}$alkyl,
wherein
each of $R_5$, $R_8$, $R_{11}$ and $R_{14}$ is independently hydrogen, $C_{1-3}$alkyl, n-butyl, i-butyl, t-butyl, $C_{1-3}$alkoxy, n-butoxy, i-butoxy, trifluoromethyl, fluoro, chloro, phenyl, phenoxy or benzyloxy,
each of $R_6$, $R_9$, $R_{12}$ and $R_{15}$ is independently hydrogen, $C_{1-3}$alkyl, $C_{1-3}$alkoxy, trifluoromethyl, fluoro, chloro, phenoxy or benzyloxy, and
each of $R_7$, $R_{10}$, $R_{13}$ and $R_{16}$ is independently hydrogen, $C_{1-2}$alkyl, $C_{1-2}$alkoxy, fluoro or chloro,
with the provisos that not more than one substituent on each of Rings A, B, C and D independently is trifluoromethyl, not more than one substituent on each of Rings A, B, C and D independently is phenoxy, and not more than one substituent on each of Rings A, B, C and D independently is benzyloxy, and
each m is independently 1, 2 or 3,
in free acid form, or in the form of an ester of δ-lactone thereof, or in salt form as appropriate.

2. The compound according to claim 1 which is (3R,5S)-(E)-3,5-dihydroxy-7-[1′,3′-dimethyl-4′-(4″-fluorophenyl)-6′-(1″-methylethyl)-1H-pyrazolo[3,4-b]pyrid-5′-yl]hept-6-enoic acid, in free acid form, or in form of an ester thereof, or in salt form.

3. A compound according to claim 1 which is selected from:
- (±)-erythro-(E)-3,5-dihydroxy-7-[1′,6′-dimethyl-4′-(4″-fluorophenyl)-3′-phenyl-1H-pyrazolo[3,4-b]pyrid-5′-yl]hept-6-enoic acid, in free acid form, or in form of an ester thereof, or in salt form;
- the compound of Formula I wherein $R_2$, $R_3$ and $R_4$ are methyl, $R_2$ is 4-fluorophenyl, X is (E)-CH=CH-and Z is a group a wherein $R_{17}$ is hydrogen, in free acid form, or in form of an ester or the δ-lactone thereof, or in salt form;
- the compound of Formula I wherein $R_1$ is isopropyl, $R_2$ is 4-fluorophenyl, $R_3$ and $R_4$ are methyl, X is (E)-CH=CH- and Z is a group a wherein $R_{17}$ is hydrogen, in free acid form, or in form of an ester thereof, or in salt form;
- the compound of Formula I wherein $R_1$ is isopropyl, $R_2$ is cyclohexyl, $R_3$ and $R_4$ are methyl, X is (E)-CH=CH- and Z is a group a wherein $R_{17}$ is hydrogen, in free acid form, or in form of an ester thereof, or in salt form.

4. A compound according to any one of claims 1 to 3, in sodium salt form.

5. A pharmaceutical composition comprising a compound according to claim 1 as appropriate in free acid form, or in the form of a physiologically-hydrolysable and -acceptable ester or a δ-lactone thereof, or in pharmaceutically acceptable salt form, together with a pharmaceutically acceptable carrier or diluent.

6. A compound according to claim 1 as appropriate in free acid form or in the form of a physiologically-hydrolysable and -acceptable ester or a δ-lactone thereof, or in pharmaceutically acceptable salt form, for use as a pharmaceutical.

7. A compound according to claim 1 as appropriate in free acid form or in the form of a physiologically-hydrolysable and -acceptable ester or a δ-lactone thereof, or in pharmaceutically

acceptable salt form, for use in inhibiting cholesterol biosynthesis or treating atherosclerosis.

8. A process for preparing a compound according to claim 1 which comprises hydrolysing a compound of Formula I in ester of δ-lactone form or esterifying or lactonising a compound of formula I in free acid, form,
and when a free carboxyl group is present, recovering the compound obtained in free acid form or in the form of a salt.

9. A process for the preparation of a compound according to claim 1 which comprises

a) when X is $-CH_2CH_2-$, $-CH_2CH_2CH_2-$, $-CH=CH-$, $-CH=CHCH_2-$ or $-CH_2CH=CH-$,
deprotecting a compound of Formula Pa
$PP-X_{aa}-CH(OPro)-CH_2-C(R_{17})(OPro)-CH_2-COOR_{aa}$ (Pa),
wherein
PP is a group of Formula

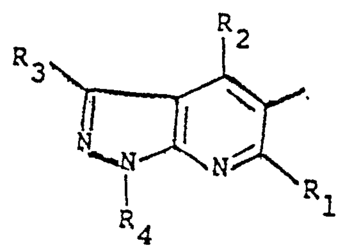

wherein $R_1$ to $R_4$ are as defined in claim 1,
$X_{aa}$ is $-CH_2CH_2-$, $-CH_2CH_2CH_2-$, $-CH=CH-$, $-CH=CHCH_2-$or$-CH_2CH=CH-$,
$R_{17}$ is as defined in claim 1,
Pro is a protecting group and
$R_{aa}$ is a radical forming an ester,

b) when $R_{18}$ is hydrogen or M,
hydrolysing a compound of Formula I in the form of an ester or lactone,

c) when X is $-(CH_2)_n-$ or (E)$-CH=CH-$ and Z is a group of Formula a wherein $R_{17}$ is hydrogen,
reducing a compound of Formula Pc
$PP-X_1-CH(OH)-CH_2-C(=O)-CH_2-COOR_{aa}$ (Pc),
wherein
PP and $R_{aa}$ are as defined above and
$X_1$ is $-(CH_2)_n-$ or (E)$-CH=CH-$,

d) when X is $-(CH_2)_n-$ or (E)$-CH=CH-$ and Z is a group of Formula a wherein $R_{17}$ is $C_{1-3}$ alkyl,
hydrolysing a compound of Formula Pd
$PP-X_1-CH(OCOR_{20})-CH_2-C(R_{17a})(OH)-CH_2-COOR_{aa}$ (Pd),
wherein
PP, $X_1$ and $R_{aa}$ are as defined above,
$R_{17a}$ is $C_{1-3}$alkyl and
$R_{20}$ is part of an ester forming group,

e) when Z is a group of Formula c,
oxidising a corresponding compound of Formula I wherein Z is a group of Formula a, or

f) esterifying or lactonising a compound of Formula I in free acid form, and when a free carboxyl group is present, recovering the compound obtained in free acid form or in the form of a salt.

10. A compound of formulae VIII, XI, XIV, XVI, XIX, XX, XXII, XXIV, XLI-XLIII, XLV-XLVII, L-LII, LVII, LXI and LXII.